# EUROPEAN PATENT APPLICATION

(11) **EP 4 371 599 A1**
(43) Date of publication of application: **22.05.2024**
(21) Application number: 21840770.8
(22) Date of filing: 08.10.2021
(51) Int. Cl.: A61M 37/00, A61K 9/00

(54) **MICRONEEDLE PATCH AND MICRONEEDLE PATCH MANUFACTURING METHOD**

(30) Priority: 15.07.2021 KR 20210093117; 15.07.2021 KR 20210093120
(71) Applicant: Feroka Inc., Seoul 04784 (KR)
(72) Inventor: LEE, Jae Joon, Seoul 02793 (KR); JEON, Yi Seul, Seoul 08799 (KR)
(74) Representative: Hoffmann Eitle
(86) International application number: PCT/KR2021/013877
(87) International publication number: WO 2023/286916

(57) **Abstract**

A microneedle patch includes a base, and a microneedle, which contains an effective material, protrudes from a surface of the base, and includes a plurality of layers, a concentration of the effective material varying along a longitudinal direction of the microneedle.

## Description

### TECHNICAL FIELD

Embodiments of the present disclosure relate to a microneedle patch and a method of manufacturing the same.

### BACKGROUND ART

Injection of a drug into a human body has traditionally been performed by using a needle syringe, but such needle-syringe injection causes great pain. Non-invasive drug injection methods have been developed to overcome this issue, but these methods have a problem in that a large amount of drug is consumed compared to the amount of actually delivered drug.

In order to find a solution to this problem, many studies have been conducted on drug delivery systems (DDSs), and these studies have made even greater advances with the development of nanotechnology.

Unlike conventional injection needles, microneedles enable painless skin penetration without injury. In addition, a certain degree of physical hardness of microneedles may be required to penetrate the stratum corneum of skin. In addition, an appropriate length of microneedles may be required for physiologically active substances to reach the epidermal or dermal layer of skin. Furthermore, in order to effectively deliver physiologically active substances in hundreds of microneedles into skin, the microneedles need to have high skin permeability and be maintained for a certain period of time until dissolution after being inserted into the skin.

Accordingly, interest in microneedles capable of delivering a precise amount of a drug and accurately setting a target position is increasing.

### DESCRIPTION OF EMBODIMENTS

### TECHNICAL PROBLEM

The present disclosure may provide a microneedle patch capable of effectively delivering a preset amount of an effective material to a target position, and a method of manufacturing the microneedle patch.

### SOLUTION TO PROBLEM

An embodiment of the present disclosure provides a microneedle patch including a base, and a microneedle, which contains an effective material, protrudes from a surface of the base, and includes a plurality of layers, a concentration of the effective material varying along a longitudinal direction of the microneedle.

The microneedle may include: a first layer having a sharpened tip arranged on one side thereof and a surface formed at another side thereof to face the base; a second layer, which is connected to the base and arranged between the base and the first layer; and a connection layer, which is arranged between the first layer and the second layer and connects the first layer to the second layer.

The connection layer may be integrally formed with the first layer by dissolving the first layer.

The connection layer may be integrally formed with the second layer by dissolving the second layer.

One surface of the connection layer, which is opposite to another surface of the connection layer connected to the first layer, may have a curvature.

One surface of the second layer facing the connection layer may have a curvature.

The one surface of the connection layer may have a plurality of curvatures.

Both sides of the curvature may be symmetrical to each other with respect to a longitudinal central axis of the microneedle.

At least one of the plurality of layers may include an in vivo degradable polymer.

The microneedle patch may further include a shaft connecting the base to the microneedle.

Another embodiment of the present disclosure provides a method of manufacturing a microneedle patch, including forming a microneedle containing an effective material, wherein the forming of the microneedle includes: forming a plurality of layers; spraying a fluid onto at least one of the plurality of layers; and connecting the plurality of layers to each other.

Other aspects, features, and advantages other than those described above will be apparent from the following drawings, claims, and detailed description.

### ADVANTAGEOUS EFFECTS OF PRESENT DISCLOSURE

In a microneedle patch according to the present disclosure, the concentration of an effective material varies along the longitudinal direction of the microneedle, and the longitudinal direction may be delivered to any one of an epidermis, a dermis, subcutaneous fat, and muscle at an appropriate concentration according to a position at which the effective material is activated.

The microneedle patch according to the present disclosure has a multi-layer structure, and thus is capable of accurately delivering the effective material to a target point. The microneedle includes a plurality of layers, and thus an effective material may be arranged in each layer. Accordingly, the effective material may be delivered to any one of an epidermis, a dermis, subcutaneous fat, and muscle at an appropriate concentration according to the position at which the effective material is activated.

The microneedle patch according to the present disclosure has a multi-layer structure, and thus the biodegradation rates of the layers may be different from each other. The effective materials of the layers of the microneedle may be activated at different points of time according to the decomposition rates of the layers.

In the microneedle patch according to the present disclosure, a connection layer has a curvature, and thus a first layer and a second layer may be easily separated in vivo from each other. Because the edge of the region where respective layers are in contact with each other is thin, the first layer and the second layer may be easily separated from each other.

In the microneedle patch according to the present disclosure, because each layer has a curvature, the surface area of each layer is increased, and accordingly, the delivery effectiveness of an effective material may be increased. A first curved surface of the connection layer, which is integrally formed with the first layer by dissolving it, increases the surface area, and a second curved surface formed in the second layer facing the first curved surface increases the lower surface area of the second layer.

Such increases in surface area due to the first curved surface and the second curved surface may increase a drug delivery area, thereby improving the drug delivery effect.

In forming a microneedle including a plurality of layers, the method of manufacturing a microneedle patch according to the present disclosure is capable of reducing the period of time required for manufacturing the microneedle patch including the microneedle and a base connected to the microneedle, by individually forming the plurality of layers and then spraying a fluid onto at least one of a pair of layers connected to each other to form a connection layer and thus adhesively connect the plurality of layers to each other, rather than sequentially forming the plurality of layers.

In addition, as the connection layer is integrally formed with a layer including an effective material by dissolving a certain region of the layer, the concentration of the effective material in the region where the connection layer is formed may be relatively low, the concentration of the effective material may vary along the longitudinal direction of the microneedle, and thus a concentration gradient may be formed.

In addition, as the concentration gradient is formed along the longitudinal direction of the microneedle, the manufactured microneedle patch may deliver the effective material to any one of an epidermis, a dermis, subcutaneous fat, and muscle at an appropriate concentration according to the position at which the effective material is activated.

### BRIEF DESCRIPTION OF DRAWINGS

FIG. 1 is a perspective view illustrating a microneedle patch according to an embodiment of the present disclosure.
FIG. 2 is a diagram illustrating a cross-section of the microneedle patch of FIG. 1.
FIG. 3 is an enlarged view of a portion of FIG. 2.
FIG. 4 is an enlarged view of a part of a microneedle patch according to another embodiment of the present disclosure.
FIG. 5 is an enlarged view of region A of FIG. 3.
FIG. 6 is an enlarged view of a portion corresponding to region A of FIG. 3 in a microneedle patch according to another embodiment of the present disclosure.
FIG. 7 is a diagram illustrating a process in which the microneedle patch of FIG. 2 is attached to the skin of a user and then a drug is delivered.
FIG. 8 is a diagram illustrating a state in which a coating layer is provided on a microneedle patch, according to an embodiment of the present disclosure.
FIG. 9 is an enlarged view of a part of a microneedle patch according to another embodiment of the present disclosure.
FIG. 10 is a diagram illustrating a process in which the microneedle patch of FIG. 9 is attached to the skin of a user and then a drug is delivered.
FIG. 11 is a diagram illustrating a state in which a coating layer is provided on a microneedle patch, according to another embodiment of the present disclosure.
FIG. 12 is a diagram illustrating a microneedle patch according to another embodiment of the present disclosure.
FIG. 13 is a diagram illustrating a microneedle patch according to another embodiment of the present disclosure.
FIG. 14 is a flowchart of a method of manufacturing a microneedle patch according to an embodiment of the present disclosure.
FIG. 15 is a flowchart of an operation of forming a microneedle, according to an embodiment of the present disclosure.
FIG. 16 is a flowchart of an operation of forming a plurality of layers, according to an embodiment of the present disclosure.
FIGS. 17 and 18 are diagrams illustrating processes of forming connection layers.

### BEST MODE

As the present disclosure allows for various changes and numerous embodiments, particular embodiments will be illustrated in the drawings and described in detail. The effects and features of the present disclosure and methods of achieving them will become clear with reference to the embodiments described in detail below with the drawings. However, the present disclosure is not limited to the embodiments disclosed below, and may be implemented in various forms.

While such terms as "first," "second," etc., are used only to distinguish one component from another, and such components must not be limited by these terms.

The singular expression also includes the plural meaning as long as it is not inconsistent with the context.

The terms "comprises," "includes," or "has" used herein specify the presence of stated features or elements, but do not preclude the presence or addition of one or more other features or elements.

It will be understood that when a layer, region, or component is referred to as being "on" another layer, region, or component, it may be directly or indirectly on the other layer, region, or component, that is, one or more intervening layers, regions, or components may be present therebetween.

When a certain embodiment may be differently implemented, specific operations may be performed differently from the sequence described herein. For example, two consecutive operations may be performed substantially at the same time, or may be performed in an order opposite to the order described herein.

For ease of description, the magnitude of components in the drawings may be exaggerated or reduced. For example, the magnitude and thickness of each component in the drawings is illustrated for ease of description, and the present disclosure is not limited to the drawings.

In the present specification, expressions such as 'front' and 'rear' may be based on the x-axis shown in the drawing, and expressions such as 'left' and 'right' may be based on the y-axis shown in the drawing, and expressions such as 'on' and 'below' may be based on the z-axis shown in the drawing.

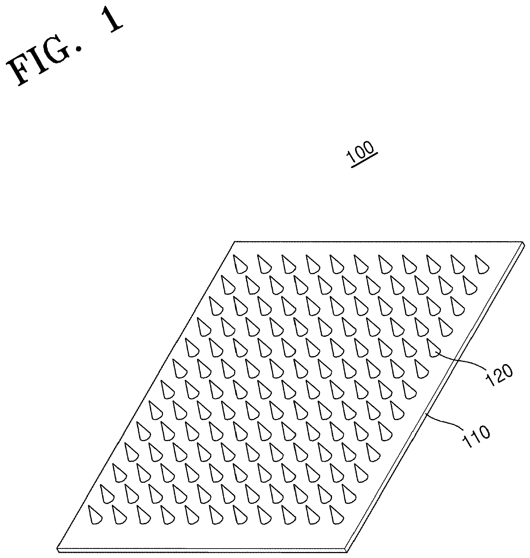

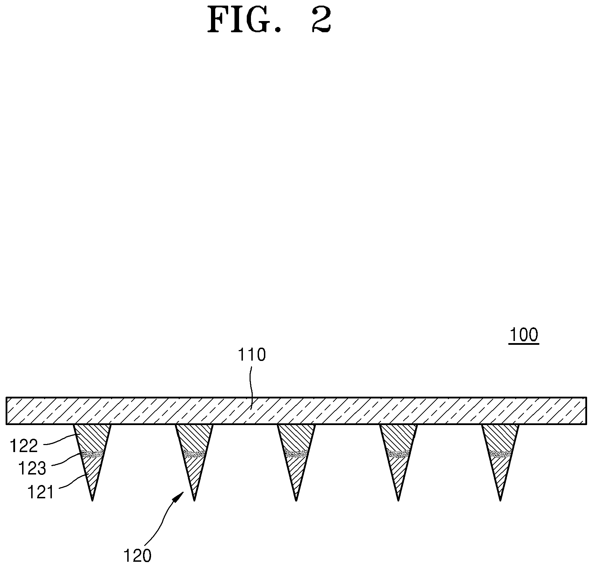

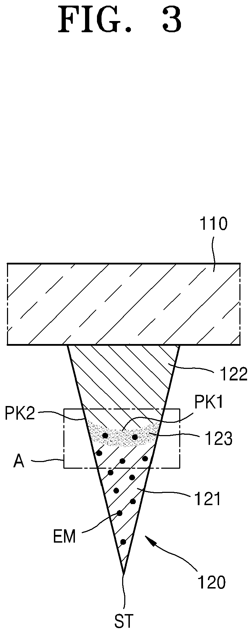

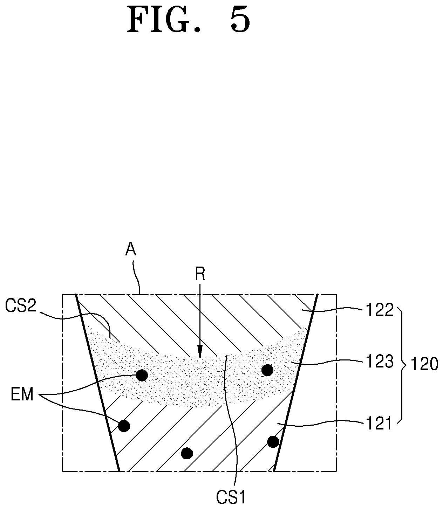

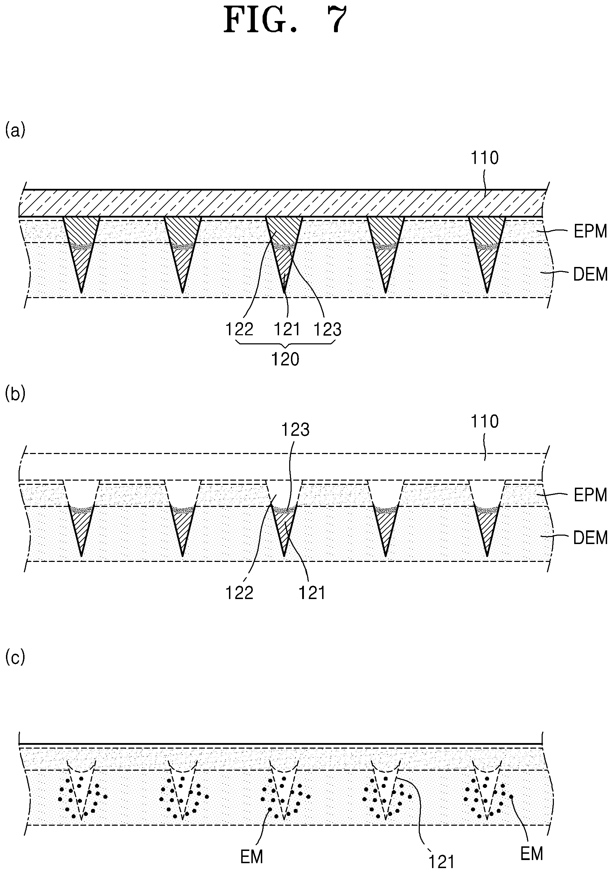

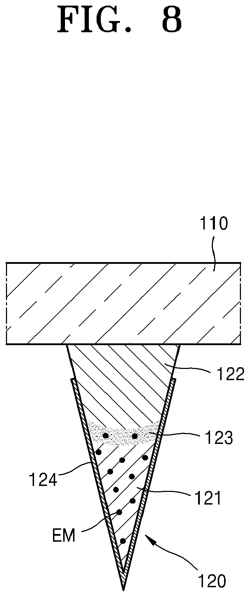

FIG. 1 is a perspective view illustrating a microneedle patch 100 according to an embodiment of the present disclosure. FIG. 2 is a diagram illustrating a cross-section of the microneedle patch 100 of FIG. 1. FIG. 3 is an enlarged view of a portion of FIG. 2. FIG. 5 is an enlarged view of region A of FIG. 3. FIG. 7 is a diagram illustrating a process in which the microneedle patch 100 of FIG. 2 is attached to the skin of a user and then a drug is delivered. FIG. 8 is a diagram illustrating a state in which a coating layer 124 is provided on a microneedle patch, according to an embodiment of the present disclosure.

Referring to FIGS. 1 to 3, 5, 7, and 8, the microneedle patch 100 according to an embodiment of the present disclosure having a multi-layer structure may include a base 110 and microneedles 120.

Referring to FIGS. 1 to 3, 7, and 8, the base 110 according to an embodiment of the present disclosure may support the microneedles 120, and may include a plurality of microneedles 120 on one surface (the lower surface in FIG. 2) thereof. The one surface of the base 110 may come into contact with skin, and the other surface of the base 110 may be exposed to the outside.

The base 110 according to an embodiment of the present disclosure may be removed after the microneedles 120 are inserted into the skin. In detail, the base 110 may be removed from the skin by a user applying a force.

In an alternative embodiment, a portion at which the base 110 and the microneedle 120 are coupled to each other first dissolves, and thus the base 110 may be removed after a certain period of time has elapsed after the microneedle patch 100 is attached to the skin.

In another alternative embodiment, the base 110 may dissolve after a long period of time has elapsed after the microneedle patch 100 is attached to the skin.

In another alternative embodiment, the base 110 to be attached to the skin of the user may be formed of a dissolvable material, and may be removed by the user applying a material for dissolution thereon, if necessary.

The base 110 according to an embodiment of the present disclosure may include any one of materials included in the microneedle 120. The base 110 may include a biodegradable material similarly to the microneedle 120.

For example, the base 110 may include the same material as that of any one of a plurality of layers of the microneedle 120.

In an alternative embodiment, the base 110 may include a physiologically active substance. After attaching the microneedle patch 100 according to an embodiment of the present disclosure to the skin, an effective drug may be effectively delivered to the patient by the physiologically active substance released from the base 110.

In addition, the base 110 and the microneedles 120 may be easily separated from each other by the physiologically active substance released from the base 110.

The base 110 according to an embodiment of the present disclosure may have a property of dissolving later than does the closest layer of the microneedle 120, i.e., a layer that is farthest away from a tip formed at the lower side of the microneedle 120, specifically, a sharpened tip ST of the microneedle 120.

Consequently, a portion of the microneedle 120, which is adjacent to the base 110, dissolves the fastest, and thus the base 110 may be easily separated from the microneedle 120.

In an alternative embodiment, the base 110 may include a water-soluble polymer. The base 110 may be formed of a water-soluble polymer and may include other additives (e.g., disaccharides, etc.). In addition, it is preferable that the base 110 does not include a drug or an effective material.

The base 110 according to an embodiment of the present disclosure may include a biocompatible material. A biocompatible material selected as a base material of the microneedle 120, which will be described below, may also be selected as a base material of the base 110.

Referring to FIGS. 3 and 5 to 8, the microneedle 120 according to an embodiment of the present disclosure contains an effective material EM and protrudes from the surface of the base 110, and may be formed to have a plurality of layers.

In the microneedle 120 according to an embodiment of the present disclosure, the concentration of the effective material EM may vary along the longitudinal direction (the vertical direction of FIG. 2), and thus a concentration gradient may be formed.

The microneedle 120 according to an embodiment of the present disclosure may be formed of a biocompatible material and an additive.

The biocompatible material may include at least any one of carboxymethyl cellulose (CMC), hyaluronic acid (HA), alginic acid, pectin, carrageenan, chondroitin sulfate, dextran sulfate, chitosan, polylysine, carboxymethyl chitin, fibrin, agarose, pullulan, polyanhydride, polyorthoester, polyetherester, polyesteramide, polybutyric acid, polyvaleric acid, polyacrylate, an ethylene-vinyl acetate polymer, acryl-substituted cellulose acetate, polyvinyl chloride, polyvinyl fluoride, polyvinyl imidazole, chlorosulphonate polyolefins, polyethylene oxide, polyvinylpyrrolidone (PVP), hydroxypropyl methylcellulose (HPMC), ethylcellulose (EC), hydroxypropyl cellulose (HPC), cyclodextrin, maltose, lactose, trehalose, cellobiose, isomaltose, turanose, and lactulose, or at least any one of a copolymer of monomers forming such polymers and cellulose.

The additive may include at least any one of trehalose, oligosaccharide, sucrose, maltose, lactose, cellobiose, HA, alginic acid, pectin, carrageenan, chondroitin sulfate, dextran sulfate, chitosan, polylysine, collagen, gelatin, carboxymethyl chitin, fibrin, agarose, PVP, polyethylene glycol (PEG), polymethacrylate, HPMC, EC, HPC, carboxymethyl cellulose, cyclodextrin, gentiobiose, cetrimide (alkyltrimethylammonium bromide), cetrimonium bromide (hexadecyltrimethylammonium bromide (CTAB)), gentian violet, benzethonium chloride, docusate sodium salt, a SPAN-type surfactant, polysorbate (Tween), sodium lauryl sulfate (sodium dodecyl sulfate (SDS)), benzalkonium chloride, and glyceryl oleate.

The term "hyaluronic acid (HA)" is used herein to encompass hyaluronic acid, hyaluronates (e.g., sodium hyaluronate, potassium hyaluronate, magnesium hyaluronate, and calcium hyaluronate), and mixtures thereof. The term "hyaluronic acid (HA)" is also used here to encompass cross-linked hyaluronic acid and/or non-cross-linked hyaluronic acid.

According to an embodiment of the present disclosure, the molecular weight of the HA is 2 kDa to 5000 kDa.

According to another embodiment of the present disclosure, the molecular weight of the HA is 100 kDa to 4500 kDa, 150 kDa to 3500 kDa, 200 kDa to 2500 kDa, 220 kDa to 1500 kDa, 240 kDa to 1000 kDa, or 240 kDa to 490 kDa.

The CMC used herein may be known CMC with various molecular weights. For example, the average molecular weight of the CMC used herein is 90,000 kDa, 250,000 kDa, or 700,000 kDa.

The disaccharides may be sucrose, lactulose, lactose, maltose, trehalose, cellobiose, or the like, and may particularly include sucrose, maltose, and trehalose.

In an alternative embodiment, the microneedle 120 may include an adhesive. The adhesive is at least one adhesive selected from the group consisting of silicone, polyurethane, HA, a physical adhesive (Gecko), polyacryl, EC, hydroxymethyl cellulose, ethylene-vinyl acetate, and polyisobutylene.

In an alternative embodiment, the microneedle 120 may further include a metal, a polymer, or an adhesive.

The microneedle 120 according to an embodiment of the present disclosure may include the effective material EM. The microneedle 120 may include the effective material EM in at least a portion thereof, and the effective material EM may be a pharmaceutically, medically, or cosmetically effective material.

For example, the effective material may include, but is not limited to, a protein/peptide medicine, and may include at least one of a hormone, a hormone analogue, an enzyme, an enzyme inhibitor, a signal transduction protein or a portion thereof, an antibody or a portion thereof, a single-chain antibody, a binding protein or a binding domain thereof, an antigen, an adherent protein, a structural protein, a regulatory protein, a toxic protein, a cytokine, a transcription regulator, a blood coagulation factor, and a vaccine.

In detail, the protein/peptide medicine may include at least one of insulin, insulin-like growth factor 1 (IGF-1), growth hormone, erythropoietin, granulocyte colony-stimulating factors (G-CSFs), granulocyte/macrophage colony-stimulating factors (GM-CSFs), interferon alpha, interferon beta, interferon gamma, interleukin-1 alpha and beta, interleukin-3, interleukin-4, interleukin-6, interleukin-2, epidermal growth factors (EGFs), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II (GHRH-II), gonadorelin, goserelin, histrelin, leuprorelin, lypressin, octreotide, oxytocin, pitressin, secretin, sincalide, terlipressin, thymopentin, thymosine, triptorelin, bivalirudin, carbetocin, cyclosporine, exedine, lanreotide, luteinizing hormone-releasing hormone (LHRH), nafarelin, parathyroid hormone, pramlintide, enfuvirtide (T-20), thymalfasin, and ziconotide.

In addition, the effective material EM may be a cosmetic material such as a skin lightening agent, a filler, a wrinkle reducing agent, or an antioxidant.

In an embodiment, the effective material EM may be colloid particles dispersed in a solvent forming the microneedle 120. The particles themselves may be the effective material EM or may include a coating material carrying the effective material EM.

The effective material EM may be intensively distributed in a partial layer of the microneedle 120. That is, the effective material EM may be at a certain height in the microneedle 120, and thus, the effective material EM may be effectively delivered.

In another embodiment, the effective material EM may be dissolved in the microneedle 120. The effective material EM may be dissolved in the base material of the microneedle 120, such as the biodegradable materials described above, to constitute the microneedle 120.

The effective material EM may be uniformly dissolved in the base material and may be intensively distributed at a certain height of the microneedle 120, like the above-described particles.

The effective material EM may be distributed in a connection layer 123, which will be described below, when the connection layer 123 is formed while dissolving at least one of a first layer 121 and a second layer 122.

Consequently, the concentration of the effective material (EM) may relatively decrease in the direction from the portion where the sharpened tip ST is formed in the microneedle 120 containing the effective material EM, to the base 110.

In an alternative embodiment, when the effective material (EM) is contained in the microneedle 120, specifically, in the second layer 122, as the connection layer 123 connects the first layer 121 to the second layer 122, the effective material EM contained in the second layer 122 may be distributed in the connection layer 123.

Consequently, the concentration of the effective material EM contained in the second layer 123 may relatively decrease in the direction from the region adjacent to the base 110 to the region adjacent to the first layer 121 in the longitudinal direction of the microneedle 120.

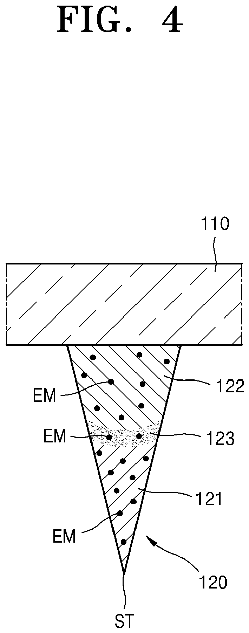

Referring to FIG. 4, the effective material (EM) according to an embodiment of the present disclosure may be distributed in the microneedle 120, specifically, in the first layer 121 and the second layer 122, respectively, and when the connection layer 123 dissolves and connects the first layer 121 and the second layer 122 to each other, the effective material EM distributed in the first layer 121 and the second layer 122 may be also distributed in the connection layer 123, and in the first layer 121, the concentration of the effective material EM may decrease in the direction from the sharpened tip ST formed at the lower end (based on the direction as illustrated in FIG. 4) of the first layer 121, to the second layer 122.

In addition, in the second layer 122, the concentration of the effective material EM may decrease in the direction away from the base 110 (in the downward direction in FIG. 4). Consequently, the concentration of the effective material (EM) may vary along the longitudinal direction (the downward direction in FIG. 4) of the microneedle 120, and a concentration gradient may be formed.

The microneedle patch 100 according to an embodiment of the present disclosure may include a plurality of effective materials (EM) in different regions thereof, respectively.

Among the plurality of microneedles 120, a first group of microneedles 120 may include a first effective material among the plurality of effective materials, and a second group of microneedles 120, which is different from the first group, may include a second effective material among the plurality of effective materials.

In an alternative embodiment, the pharmaceutically, medically, or cosmetically effective material EM may be coated on the microneedle 120. The effective materials may be coated on the entire microneedle 120 or only a portion of the microneedle 120.

In an alternative embodiment, in the microneedle 120, the first effective material may be coated on a portion of a coating layer, and the second effective material may be coated on another portion of the coating layer.

The appearance of the microneedle 120 according to an embodiment of the present disclosure may have various shapes. The microneedle 120 may have a conical shape. For example, the microneedle 120 may have a conical shape or a polygonal shape such as a triangular pyramid shape or a quadrangular pyramid shape.

The microneedle 120 may have a layered structure. The microneedle 120 may have a plurality of stacked layers. The number of layers constituting the microneedle 120 is not limited to a certain number.

Referring to FIGS. 2 and 3, the microneedle 120 according to an embodiment of the present disclosure may include the first layer 121, the second layer 122, and the connection layer 123.

In the first layer 121 according to an embodiment of the present disclosure, the sharpened tip ST may be arranged at one side (the lower side of the first layer 121 in FIG. 3), and a surface facing the base 110 may be formed at the other side.

According to an embodiment of the present disclosure, in the first layer 121, the surface facing the side (the lower side in FIG. 3) where the tip ST is formed may connected to the connection layer 123 and may be integrally formed with the connection layer 123.

In detail, a fluid may be sprayed from an external nozzle onto one surface (the upper surface in FIG. 3) of the first layer 121 facing the base 110, and may then dissolve the surface (the upper surface in FIG. 3) of the first layer 121 to form the connection layer 123. The connection layer 123 may be integrally formed with the first layer 121.

The microneedle 120 according to an embodiment of the present disclosure may have a curvature in a region where adjacent layers are in contact with each other.

The microneedle 120 may have a curvature formed in a region in which the first layer 121 and the connection layer 123 are connected to each other, and may have a curvature in a region in which the second layer 122 and the connection layer 123 are connected to each other.

The layer of the microneedle 120 according to an embodiment of the present disclosure may have a curvature downwardly convex toward the tip. In a region in which the connection layer 123, which is integrally formed with an upper region (based on the direction as illustrated in FIG. 3) of the first layer 121 by dissolving the first layer 121, is in contact with the second layer 122, a portion adjacent to the tip may have a downwardly convex shape.

In an alternative embodiment, in a region in which the connection layer 123, which is integrally formed with a lower region (based on the direction as illustrated in FIG. 3) of the second layer 122 by dissolving the second layer 122, is in contact with the first layer 121, a portion adjacent to the tip may have a downwardly convex shape.

The curvature may be formed to have both sides symmetrical to each other with respect to the longitudinal direction (the vertical direction in FIG. 5) of the microneedle 120.

Referring to FIGS. 2 and 5, the effective material EM may be included in the first layer 121 according to an embodiment of the present disclosure. In order to target a drug to a position slightly deep from a skin surface, the effective material EM may be contained in the first layer 121. For example, in order to deliver the effective material EM to a dermis DEM, a subcutaneous fat layer, or muscle, the effective material EM may be included in the first layer 121.

Referring to FIGS. 2, 3, and 5, in the first layer 121 according to an embodiment of the present disclosure, the connection layer 123 is connected to one side (the upper side in FIG. 3) opposite to the sharpened tip ST, and may be integrally formed with the first layer 121.

Referring to FIG. 5, the connection layer 123 may be formed by spraying a fluid to one side of the first layer 121, and may have a certain curvature and form a first curved surface CS1.

The connection layer 123 may be formed by spraying the fluid onto one surface of the first layer 121 manufactured in a mold. The fluid may include moisture. The fluid may dissolve a certain region on one surface of the first layer 121 to form the connection layer 123.

The connection layer 123, which is connected to and thus formed integrally with the first layer 121 after dissolving the upper surface of the first layer 121, may be convex toward the other side opposite to the side connected to the first layer 121.

As the certain region of the first layer 121 is dissolved and is integrally formed with the connection layer 123, the concentration of the effective material EM contained in the first layer 121 relatively decrease in the direction from the sharpened tip ST to the connection layer 123.

Consequently, the concentration of the effective material EM contained in the first layer 121 may be set to vary along the longitudinal direction (the vertical direction in FIG. 3) of the microneedle 120, specifically, of the first layer 121, and in detail, the concentration of the effective material EM may relatively decrease in the direction away from the sharpened tip ST.

Referring to FIGS. 3 and 5, the second layer 122 according to an embodiment of the present disclosure may be connected to the base 110 and may be arranged between the base 110 and the first layer 121.

The other surface (the lower surface in FIG. 3) of the second layer 122, which faces the surface (the upper surface in FIG. 3) of the second layer 122, which is connected to the base 110, may be connected to the connection layer 123.

The second layer 122 may be formed by injecting a base material into the mold and drying the mold. The second layer 122 according to an embodiment of the present disclosure may be in contact with and connected to the connection layer 123 while the connection layer 123 is connected to the first layer 121.

Referring to FIG. 5, one surface of the connection layer 123, which faces the second layer 122, has the first curved surface CS1 with a certain curvature, and one surface of the second layer 122, which faces the connection layer 123 having the first curved surface CS1, may have a second curved surface CS2 with a certain curvature so as to be convex toward the connection layer 123.

The first curved surface CS1 and the second curved surface CS2 may have the same radius of curvature. Because one surface of the second layer 122 is formed as the second curved surface CS2, when the second layer 122 is in contact with and connected to an upper portion of the connection layer 123, which is integrally formed with the first layer 121, a connection area at the edge is relatively smaller than a connection area at the center in the longitudinal direction, and thus, the first layer 121 and the second layer 123 may be easily separated from each other.

In the microneedle 120 according to an embodiment of the present disclosure, the connection layer 123 is arranged on the first layer 121 and is connected to the second layer 122, but the present disclosure is not limited thereto, and various modifications are possible, for example, the connection layer 123 may be formed by dissolving a certain region of the upper surface of the first layer 121 and dissolving a certain region of the lower surface of the second layer 122 facing the connection layer 123.

In this case, the connection layer 123 connected to the second layer 122 may be manufactured by spraying a fluid onto one surface of the second layer 122 manufactured in the mold. The fluid may include moisture. The fluid may dissolve a certain region on one surface of the second layer 122 to form the connection layer 123.

Referring to FIG. 3, according to an embodiment of the present disclosure, the first layer 121 and the connection layer 123, which is integrally formed with the first layer 121 by dissolving the first layer 121 and is connected to the first layer 121, contain the effective material EM, but the present disclosure is not limited thereto, and various modifications are possible, for example, the first layer 121, the second layer 122, and the connection layer 123 may contain the effective material EM as illustrated in FIG. 4.

Referring to FIG. 4, in an alternative embodiment, the second layer 122 may contain the effective material EM, and in detail, the effective material EM may be included in the second layer 122 so as to deliver the effective material EM to an epidermis EPM or a portion of the dermis DEM close to the epidermis EPM.

In an alternative embodiment, the same effective material EM may be included in each of the first layer 121 and the second layer 122. In order to deliver a drug to a portion with a wide range of depth, drugs including the same effective material EM may be included in the first layer 121 and the second layer 122, respectively.

In an alternative embodiment, the first layer 121 and the second layer 122 may include different effective materials EM. For example, the first effective material EM included the first layer 121 may be a drug targeted to the dermis DEM, and the second effective material EM included in the second layer 122 may be a drug targeted to the epidermis EPM.

In this case, the delivery rates of the first effective material EM and the second effective material EM may be adjusted by adjusting the biodegradation rates of the first layer 121 and the second layer 122.

According to an embodiment of the present disclosure, the first layer 121 and the second layer 122 may have different biodegradation rates after insertion into the skin. Any one of the first layer 121 and the second layer 122 may have a biodegradation rate greater than that of another. The biodegradation rates of the first layer 121 and the second layer 122 may depend on the types and amounts of the biocompatible materials constituting the layers.

For example, when the biodegradation rate of the first layer 121 is greater than the biodegradation rate of the second layer 122, the effective material EM may be rapidly delivered to the dermis DEM. When the biodegradation rate of the second layer 122 is greater than the biodegradation rate of the first layer 121, the effective material EM may be rapidly delivered to the epidermis EPM. In addition, when the biodegradation rate of the second layer 122 is greater than the biodegradation rate of the first layer 121, the second layer 122 may rapidly biodegrade, thus the base 110 may be rapidly removed, and the effective material EM included in the first layer 121 may be released into the skin.

Referring to FIG. 4, because the connection layer 123 is connected to the second layer 122, when the effective material EM is contained in the second layer 122, the concentration of the effective material EM contained in the second layer 122 relatively decreases in the direction from one side (the upper side in FIG. 4) facing the base 110 to the first layer 121.

Consequently, the concentration of the effective material EM contained in the second layer 122 may be set to vary along the longitudinal direction (based on the direction as illustrated in FIG. 3) of the microneedle 120, specifically, of the second layer 122, and in detail, the concentration of the effective material EM may relatively decrease in the direction away from the base 110.

According to an embodiment of the present disclosure, the layers of the microneedle 120, i.e., the first layer 121 and the second layer 122, may have different stiffnesses. For example, when the first layer 121 has a stiffness greater than that of the second layer 122, the first layer 121 may easily penetrate the skin. As the first layer 121 rapidly biodegrades, the pain in the skin may be minimized.

Referring to FIGS. 2, 3, and 5 to 8, the connection layer 123 according to an embodiment of the present disclosure may be arranged between the first layer 121 and the second layer 122, and may connect the first layer 121 to the second layer 122.

Referring to FIG. 3, the connection layer 123 according to an embodiment of the present disclosure may be integrally formed with at least one of the first layer 121 and the second layer 122, and may have a certain curvature in a connected region.

Referring to FIG. 3, with respect to a region in which the connection layer 123 and the second layer 122 are connected to each other, the microneedle 120 may have a first point PK1 at the center thereof in the longitudinal direction, and a first height between the sharpened tip ST and the first point PK1.

Meanwhile, with respect to the region in which the connection layer 123 and the second layer 122 are connected to each other, the microneedle 120 may have a second point PK2 at an outer side thereof in the radial direction, and a second height between the sharpened tip ST and the second point PK2. The first height may be less than the second height.

In an alternative embodiment, the height of the connection layer 123, which is integrally formed with and connected to the first layer 121, i.e., the distance between the sharpened tip ST and the connection layer 123, may increase in the direction from the center in the longitudinal direction of the microneedle 120 to the outer periphery in the radial direction.

The connection layer 123 according to an embodiment of the present disclosure may be formed by spraying a fluid onto at least one of the first layer 121 and the second layer 122.

In detail, the connection layer 123 may be formed by the fluid, which is sprayed onto and then dissolves one surface of at least one (e.g., the first layer 121) of the first layer 121 and the second layer 122.

The connection layer 123 may be formed while dissolving one surface of the at least one layer, and then be in contact with another layer (e.g., the second layer 122), thereby connecting the first layer 121 and the second layer 122 to each other.

The fluid forming the connection layer 123 may include moisture. However, the present disclosure is not limited thereto, and various modifications are possible, for example, the fluid may include various materials that dissolve the first layer 121 or the second layer 122, without departing from the spirit and scope of the present disclosure.

The connection layer 123 according to an embodiment of the present disclosure may be formed in a certain region of the first layer 121 or the second layer 122, and for example, when the connection layer 123 is connected to one surface of the first layer 121, the side of the connection layer 123 opposite to another side connected to the first layer 121 may be adhesive and thus be in contact with and connected to the second layer 122.

Referring to FIGS. 3 and 5, the connection layer 123 according to an embodiment of the present disclosure may dissolve the upper surface (based on the direction as illustrated in FIG. 3) of the first layer 121 to be integrally formed with the upper surface of the first layer 121, and the other surface opposite to the surface of the connection layer 123 connected to the first layer 121 may be connected to the second layer 122.

Referring to FIG. 5, the outer circumferential surface (the upper surface in FIG. 5) of the connection layer 123 may have a certain curvature. In detail, the outer circumferential surface of the connection layer 123 may be formed to be convex toward the sharpened tip ST of the first layer 121, and the first curved surface CS1 may be provided thereon.

One surface (the lower surface in FIG. 5) of the second layer 122 facing the first curved surface CS1 formed on the connection layer 123 may have a certain curvature and be connected to the first curved surface CS1, and may have the second curved surface CS2 formed to be convex toward the connection layer 123 to correspond to the shape of the first curved surface CS1.

According to an embodiment of the present disclosure, the curvature of the first curved surface CS1 of the connection layer 123 and the curvature of the second curved surface CS2 of the second layer 122 may be substantially the same.

The connection layer 123 according to an embodiment of the present disclosure may be in contact with the second curved surface CS2 formed on the second layer 122, and may be integrally formed with the second layer 122 by dissolving the second layer 122. Consequently, the connection layer 123 may connect the first layer 121 to the second layer 122.

Referring to FIGS. 3 and 5, because the connection layer 123 according to an embodiment of the present disclosure has the curvature and connects the first layer 121 to the second layer 122, the microneedle 120 may have a layered structure, and the first layer 121 and the second layer 122 may be easily separated from each other.

A region in which the connection layer 123 and the second layer 122 are connected to each other may be thinly formed at an edge of the microneedle 120. In detail, the distance between the outer surface of the connection layer 123 and the first curved surface CS1 may be relatively short.

When the microneedle 120 is inserted into the skin, the outer surface begins to biodegrade, and because the connection layer 123, which is integrally connected to the first layer 121 by dissolving it, is easily separated by the first curved surface CS1, the first layer 121 and the second layer 122 may be easily separated from each other.

Referring to FIGS. 3 and 5, the connection layer 123 according to an embodiment of the present disclosure may be integrally formed with the first layer 121 in a certain region by dissolving the first layer 121.

Accordingly, the effective material EM contained in the first layer 121 may be also distributed in the connection layer 123, and the concentration of the effective material EM in the connection layer 123 may be relatively less than the concentration of the effective material EM in the first layer 121.

That is, the concentration of the effective material EM may vary along the longitudinal direction (the downward direction in FIG. 3) of the microneedle 120, and a concentration gradient may be formed. Accordingly, a drug may be injected into a position to which the microneedle 120 is inserted and targeted, in a desired concentration.

In addition, as the concentration of the effective material (EM) in the connection layer 123 relatively decreases, the concentration of the targeted drug may be differently set and controlled according to a skin depth of the patient.

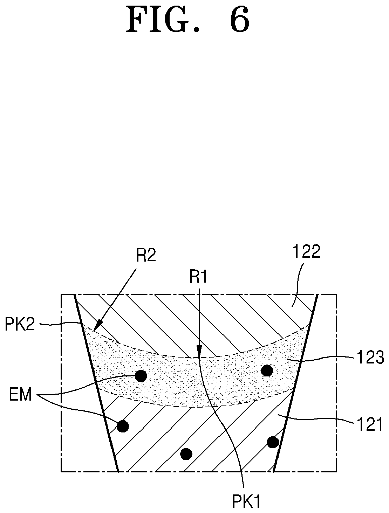

Referring to FIG. 6, the connection layer 123 may be formed to have different curvatures at regions in contact with the second layer 122, respectively.

In detail, the connection layer 123 may have a first curvature at the first point PK1, which is closer to the sharpened tip ST formed in the first layer 121 with respect to the central axis in the longitudinal direction of the microneedle 120, and may have a second curvature different from the first curvature at the second point PK2 positioned at the outside thereof.

The first curvature may be less than the second curvature. That is, the radius of curvature of the connection layer 123 may be high at the center of the microneedle 120 in the longitudinal direction, and may decrease toward the outer side.

In the case where a material, which has a high viscosity and greatly shrinks when dried, is used as the base material of the first layer 121, the first layer 121 may greatly shrink in a drying process in a state of being strongly attached to the surface of the mold due to the viscosity of the base material.

The connection layer 123 may be formed on a certain region of the first layer 121 by dissolving the first layer 121, and the connection layer 123 may also have a certain curvature according to the curvature of the first layer 121. Because the curvature at the first point PK1, which is on the central axis of the microneedle 120, is greater than the curvature at the second point PK2, the first layer 121 and the second layer 122 may be inserted to a deep position.

Furthermore, in the outer side of the microneedle 120, the thickness of the connection layer 123 integrally formed with and connected to the first layer 121 may be low in the vicinity of the second point PK2, thus the first layer 121 and the second layer 122 may be easily separated from each other, and accordingly, the drug delivery effectiveness may be increased.

Referring to FIGS. 3, 5, and 8, as the connection layer 123 according to an embodiment of the present disclosure is integrally formed with and connected to the first layer 121 by dissolving a certain region of the first layer 121, the connection layer 123 may contain the effective material EM contained in the first layer 121.

The connection layer 123 according to an embodiment of the present disclosure may be connected to the first layer 121 by dissolving the first layer 121, and consequently, may have the concentration of the effective material EM different from that of the first layer 121.

In detail, the connection layer 123 according to an embodiment of the present disclosure may include moisture, and thus, the concentration of the effective material EM in the connection layer 123 may be less than the concentration of the effective material EM contained in the first layer 121.

In other words, the concentration of the effective material EM may relatively decrease in the direction from the sharpened tip ST of the first layer 121 to the connection layer 123 (in the direction from the lower side to the upper side in FIG. 3).

That is, the concentration of the effective material EM may be set to vary along the longitudinal direction of the microneedle 120, and the concentration of a delivered drug may be differently set and controlled according to a skin depth of the patient to which the microneedle 120 penetrates.

In an alternative embodiment, referring to FIG. 4, the effective material EM may be distributed in the microneedle 120, specifically, the first layer 121 and the second layer 122, respectively, and when the connection layer 123 is connected to the first layer 121 and the second layer 122 by dissolving them, the effective material EM distributed in the first layer 121 and the second layer 122 may be distributed in the connection layer 123.

In this case, the concentration of the effective material EM in the first layer 121 may decrease in the direction from the sharpened tip ST formed at the lower end (based on the direction illustrated in FIG. 4) thereof, to the second layer 122, and the concentration of the effective material EM in the second layer 122 may decrease in the direction away from the base 110 (in the downward direction in FIG. 4).

Consequently, the concentration of the effective material (EM) may vary along the longitudinal direction (the vertical direction in FIG. 4) of the microneedle 120, and a concentration gradient may be formed.

FIG. 7 is a diagram illustrating a process in which the microneedle patch 100 of FIG. 2 is attached to deliver a drug, wherein the drug may be delivered as the microneedle patch 100 is attached to skin and then the layers of the microneedle 120 biodegrade.

Although FIG. 3 illustrates that the effective material EM is included in the first layer 121 and then delivered to the dermis DEM, the effective material EM may be included in the second layer 122 as illustrated in FIG. 4 and then delivered to the epidermis EPM.

Referring to (a) of FIG. 7, the microneedle patch 100 is attached to the skin. The microneedle 120 is inserted into the skin, and then the base 110 covers the top of the skin.

Referring to (b) of FIG. 7, the microneedle 120 may biodegrade within the skin. The microneedle 120 may be inserted into the skin, and then the base 110 may cover the top of the skin.

Referring to (c) of FIG. 7, the effective material EM may be released from the microneedle 120. When the first layer 121 begins to biodegrade, the effective material EM included therein may be delivered to the dermis DEM.

Referring to FIG. 8, the microneedle 120 according to an embodiment of the present disclosure may include the first layer 121, the second layer 122, and the connection layer 123, and the coating layer 124 may be arranged on the outer side of the microneedle 120.

The coating layer 124 according to an embodiment of the present disclosure may be formed by forming the first layer 121, the second layer 122, and the connection layer 123 and then dipping them in a coating solution. The coating layer 124 may be formed of a biocompatible polymer. The coating layer 124 may decompose after inserted into the skin.

In an alternative embodiment, the coating layer 124 may be formed of a biocompatible polymer. The coating layer 124 may decompose when inserted into the skin.

In an alternative embodiment, the coating layer 124 may include a physiologically active substance. When the coating layer 124 is inserted into the skin, the coating layer 124 may be activated first before the effective material EM is injected, and thus, the delivery effectiveness the effective material EM may be increased.

In an alternative embodiment, the coating layer 124 may be formed of a material having a high biodegradation rate. The coating layer 124 may be formed of a material having a biodegradation rate greater than those of the first layer 121, the second layer 122, and the connection layer 123, and thus, the in vivo decomposition rate of the coating layer 124 may be greater than those of the first layer 121, the second layer 122, and the connection layer 123.

In an alternative embodiment, the coating layer 124 may be formed of a material having a low biodegradation rate. The coating layer 124 may be formed of a material having a biodegradation rate less than those of the first layer 121, the second layer 122, and the connection layer 123, and thus, the in vivo decomposition rate of the coating layer 124 may be less than those of the first layer 121, the second layer 122, and the connection layer 123.

According to an embodiment of the present disclosure, after the microneedle 120 is inserted into the skin, a drug may be delivered after a certain period of time has elapsed, and thus the effective material EM may be delivered at a preferred appropriate point of time.

In an alternative embodiment, the coating layer 124 may increase the stiffness of the microneedle 120. Because the coating layer 124 covers the outer side of the connection layer 123 connected to the first layer 121 and the second layer 122, the first layer 121 and the second layer 122 may be prevented from being separated from each other when the microneedle 120 is inserted into the skin.

A method of manufacturing the microneedle patch 100 according to an embodiment of the present disclosure will be described.

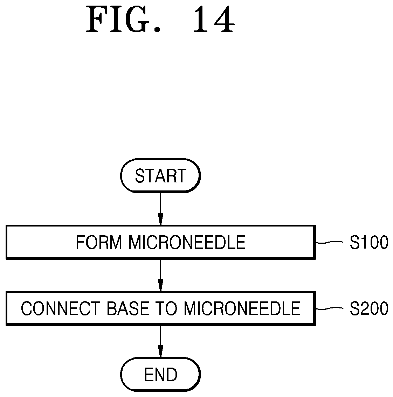

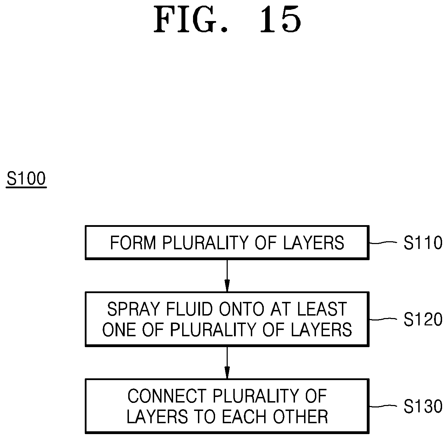

FIG. 14 is a flowchart of a method of manufacturing the microneedle patch 100 according to an embodiment of the present disclosure. FIG. 15 is a flowchart of operation S100 of forming the microneedle 120, according to an embodiment of the present disclosure.

Referring to FIGS. 14 and 15, the method of manufacturing the microneedle patch 100 according to an embodiment of the present disclosure may include forming a microneedle (S100) and connecting a base to the microneedle (S200).

Referring to FIG. 15, the forming of the microneedle (S100) may include forming a plurality of layers (S110), spraying a fluid onto at least one of the plurality of layers (S120), and connecting the plurality of layers to each other (S130).

The microneedle patch 100 according to an embodiment of the present disclosure may be manufactured by forming the microneedle 120 and then connecting the microneedle 120 to the base 110.

The microneedle 120 according to an embodiment of the present disclosure may include a plurality of layers, which may be independently formed.

In an embodiment, the first layer 121 and the second layer 122 are first formed, and the connection layer 123 is formed by connecting the first layer 121 to the second layer, but the present disclosure is not limited thereto, and various modifications are possible, for example, three or more layers are provided and the connection layer 123 is formed between every adjacent layers.

In the forming of the plurality of layers (S110), the plurality of layers may be formed. The plurality of layers may be formed by injecting base materials into different molds and drying the base materials.

In detail, the first layer 121 may be formed by injecting and drying a first base material into the mold, and the second layer 122 may be formed by injecting and drying a second base material into the mold.

The sharpened tip ST may be formed at one side of the first layer 121, and the cross-sectional area of the mold with respect to the central axis in the longitudinal direction may decrease in a preset direction to form the sharpened tip ST.

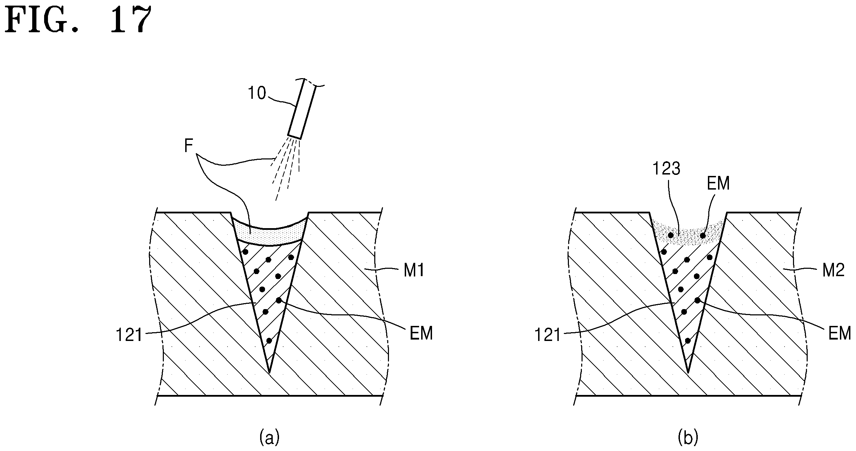

The first base material may include a biocompatible polymer or an adhesive. In an alternative embodiment, the first base material may contain the effective material EM. After the first layer 121 is formed, a fluid may be sprayed from a nozzle 10 (see FIG. 17) installed outside the first layer 121.

In detail, the fluid may be sprayed onto one surface of the first layer 121 facing the second layer 122, and the one surface may be then partially dissolved. As the fluid is sprayed onto the surface of the first layer 121, a certain region of the first layer 121 may be dissolved to form the connection layer 123, and the second layer 122 may be connected to the connection layer 123 such that the first layer 121 and the second layer 122 are connected to each other.

One surface of the connection layer 123 facing the second layer 122 may be formed to be downwardly convex toward the first layer 121 due to the viscosity and drying-induced shrinkage of the first base material and the fluid.

One surface of the second layer 122 facing the connection layer 123 may be formed to be downwardly convex toward the connection layer 123 due to the viscosity and drying-induced shrinkage of the second base material and the fluid.

The fluid for forming the connection layer 123 may be sprayed onto any one of the first layer 121 and the second layer 122, then dissolve a certain region of the first layer 121 or the second layer 122 to be integrally formed with the first layer 121 or the second layer 122 and connect the first layer 121 to the second layer 122.

The fluid may include moisture, and because the effective material EM is contained in the first layer 121, the concentration of the effective material EM in the connection layer 123 may be less than the concentration of the effective material EM in the first layer 121.

In detail, the concentration of the effective material EM may relatively decrease in the direction from the sharpened tip ST formed on the first layer 121, to the connection layer 123.

In an alternative embodiment, the effective material EM may be contained in the second layer 122, and the concentration of the effective material EM in the connection layer 123 may be less than the concentration of the effective material EM in the second layer 122.

Consequently, the concentration of the effective material EM in the second layer 122 may relatively decrease in the direction from the base 110 to the connection layer 123.

When the microneedle 120 according to an embodiment of the present disclosure is formed, the microneedle patch 100 may be manufactured by attaching one side of the microneedle 120 to the base 110.

In an alternative embodiment, a third layer may be formed in addition to the first layer 121 and the second layer 122, the connection layer 123 may be formed by spraying a fluid onto one of the second layer 122 and the third layer, the microneedle 120 including the first layer 121, the second layer 122, and the third layer may be manufactured by connecting the second layer 122 to the third layer, and the microneedle patch 100 may be manufactured by connecting the microneedle 120 to the base 110.

In an alternative embodiment, a shaft may be formed by injecting a third base material into another mold, and a plurality of microneedles 120 may be arranged on one surface of the base 110 by aligning and attaching the shaft with the second layer 122.

Hereinafter, the configuration, operation principle, and effects of the microneedle patch 100 according to another embodiment of the present disclosure will be described.

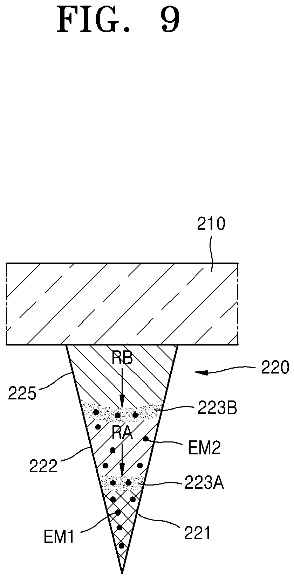

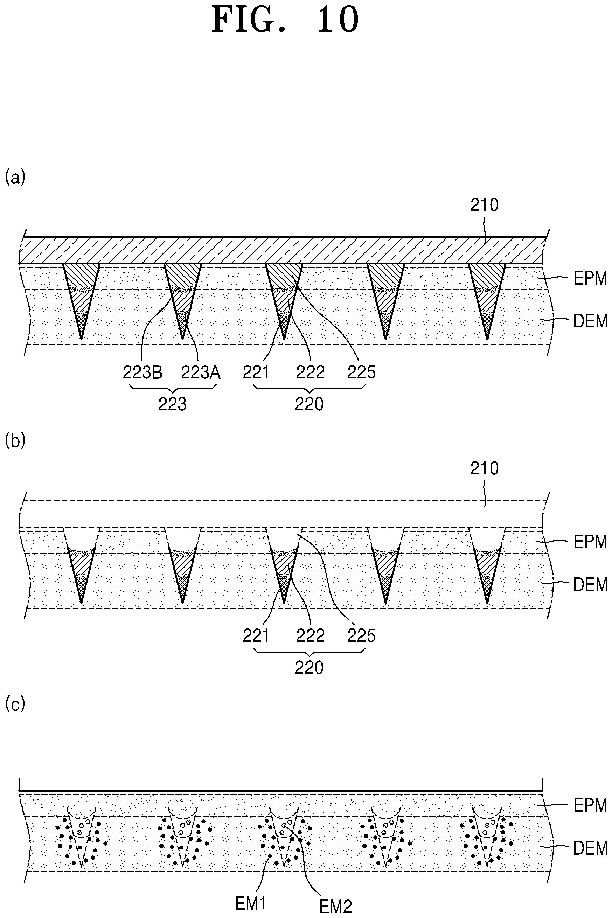

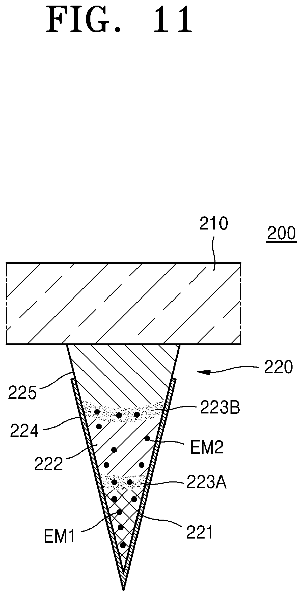

FIG. 9 is an enlarged view of a part of a microneedle patch according to another embodiment of the present disclosure. FIG. 10 is a diagram illustrating a process in which the microneedle patch of FIG. 9 is attached to the skin of a user and then a drug is delivered. FIG. 11 is a diagram illustrating a state in which a coating layer 224 is provided on a microneedle patch, according to another embodiment of the present disclosure.

A microneedle patch 200 according to another embodiment of the present disclosure may include a base 210 and a microneedle 220.

Referring to FIG. 10, the microneedle 220 according to an embodiment of the present disclosure may include a first layer 221, a second layer 222, a third layer 225, and connection layers 223, 223A, and 223B.

The third layer 225 may be formed by injecting a third base material into a mold and drying the third base material, and the second layer 222 and the third layer 225 may be connected to each other by spraying a fluid F (see FIG. 17) onto at least one of the second layer 222 and the third layer 225 to form the connection layer 223B.

One surface (the lower surface in FIG. 9) of the second layer 222 connected to the connection layer 223A connected to the first layer 221 may have a first curvature RA, and one surface (the lower surface in FIG. 9) of the third layer 225 connected to the connection layer 223B connected to the second layer 222 may have a second curvature RB.

The first curvature RA and the second curvature RB may be equal to each other. However, the present disclosure is not limited thereto, and various modifications are possible, for example, the first curvature RA and the second curvature RB may be different from each other.

Referring to FIG. 9, in the microneedle patch 200 according to another embodiment of the present disclosure, the first layer 221 and the second layer 222 may contain different effective materials. The first layer 221 may contain a first effective material EM1, and the second layer 222 may contain a second effective material EM2.

Consequently, the first effective material EM1 and the second effective material EM2 may be contained in the connection layer 223A in which the first layer 221 and the second layer 222 are connected to each other. The connection layer 223A may include moisture and may be integrally formed with at least one of the first layer 221 or the second layer 222 by dissolving it, and thus the concentration of each of the first and second effective materials EM1 and EM2 in the connection layer 223A may be reduced.

In detail, the concentration of the first effective material EM1 may relatively decrease in the direction from the sharpened tip ST of the first layer 221 to the second layer 222, and the concentration of the second effective material EM2 may relatively decrease in the downward direction (based on the direction as illustrated in FIG. 9).

Although not illustrated in FIG. 9, the effective material may also be contained in the third layer 225, and the concentration of the effective material may vary along the longitudinal direction of the third layer 225 due to the connection layers 223.

FIG. 10 illustrates a process in which the microneedle patch 200 of FIG. 9 is attached to the skin of a patient and then a drug is delivered, wherein the first effective material EM1 is included in the first layer 221, the second effective material EM2 is included in the second layer 222, and positions to which the effective materials EM1 and EM2 are to be delivered may depend on the positions of the effective materials EM1 and EM2.

In addition, the connection layer 223, which includes moisture and is integrally formed with at least one of the first layer 221 or the second layer 222 by dissolving it, causes the concentrations of the effective materials EM to vary along the longitudinal direction (the vertical direction in FIG. 9) of the microneedle 220 according to the positions to which the effective materials EM are to be delivered.

Referring to (a) of FIG. 10, the microneedle patch 200 is attached to the skin. The microneedle 220 is inserted into the skin, and then the base 210 covers the top of the skin.

Referring to (b) of FIG. 10, the microneedle 220 biodegrades within the skin. When the third layer 225 biodegrades first, the base 210 may be easily separated from the third layer 225.

Referring to (c) of FIG. 10, the effective materials EM1 and EM2 may be released from the microneedle 220. When the first layer 221 begins to biodegrade, the first effective material EM1 included therein may be delivered to the dermis DEM, and when the second layer 222 begins to biodegrade, the second effective material EM2 included therein may be delivered to the dermis DEM.

In this case, the first effective material EM1 and the second effective material EM2 may interact with each other to enhance the pharmacological effect in the dermis DEM.

Although FIG. 10 illustrates an example in which all of the effective materials EM1 and EM2 are delivered to the dermis DEM, the present disclosure is not limited thereto, and the effective materials EM1 and EM2 may be delivered to only the epidermis EPM or both the epidermis EPM and the dermis DEM.

Referring to FIG. 11, the microneedle patch 200 according to another embodiment of the present disclosure may include the first layer 221, the second layer 222, the third layer 225, and the connection layers 223A and 223B, and the coating layer 224 may be arranged on the outer side of the microneedle 220.

The coating layer 224 may be formed by forming the first layer 221, the second layer 222, the third layer 225, and the connection layers 223A and 223B, and then dipping them into a coating solution. The coating layer 224 may be formed of a biocompatible polymer. The coating layer 224 may decompose after inserted into the skin.

In an alternative embodiment, the coating layer 224 may be formed of a biocompatible polymer. The coating layer 224 may decompose when inserted into the skin.

In an alternative embodiment, the coating layer 224 may include a physiologically active substance. When the coating layer 224 is inserted into the skin, the coating layer 224 may be activated first before the effective materials EM1 and EM2 is injected, and thus, the delivery effectiveness the effective materials EM1 and EM2 may be increased.

In an alternative embodiment, the coating layer 224 may be formed of a material having a high biodegradation rate. The coating layer 224 may be formed of a material having a biodegradation rate greater than those of the first layer 221, the second layer 222, the third layer 225 and the connection layers 223, and thus, the in vivo decomposition rate of the coating layer 224 may be greater than those of the first layer 221, the second layer 222, the third layer 225 and the connection layers 223.

In an alternative embodiment, the coating layer 224 may be formed of a material having a low biodegradation rate. The coating layer 224 may be formed of a material having a biodegradation rate less than those of the first layer 221, the second layer 222, the third layer 225 and the connection layers 223, and thus, the in vivo decomposition rate of the coating layer 224 may be less than those of the first layer 221, the second layer 222, the third layer 225 and the connection layers 223.

According to an embodiment of the present disclosure, after the microneedle 220 is inserted into the skin, a drug may be delivered after a certain period of time has elapsed, and thus the effective materials EM1 and EM2 may be delivered at a preferred appropriate point of time.

In an alternative embodiment, the coating layer 224 may increase the stiffness of the microneedle 220. Because the coating layer 224 covers the outer side of the connection layers 223 connecting the first layer 221 to the second layer 222, and the second layer 222 to the third layer 225, respectively, the separation of the first layer 221 from the second layer 222 and the separation of the second layer 222 from the third layer 225 may be prevented when the microneedle 220 is inserted into the skin.

The microneedle patch 200 according to the present disclosure has a multi-layer structure, and thus may accurately deliver the effective materials EM1 and EM2 to a target point. Because the microneedle 220 includes the plurality of layers, the effective materials EM1 and EM2 may be included in the respective layers. Accordingly, the microneedle patch 200 may deliver the effective materials EM1 and EM2 to any one of the epidermis EPM, the dermis DEM, subcutaneous fat, and muscle, according to positions at which the effective materials EM1 and EM2 are activated.

Because the microneedle patch 200 according to the present disclosure has a multi-layer structure, the biodegradation rates of the layers may be different from each other. The effective materials EM1 and EM2 of the layers of the microneedle 220 may be activated at different points of time according to the decomposition rates of the layers.

In the microneedle patch 200 according to the present disclosure, as each of the connection layers 223 connects different layers to each other by dissolving their certain regions, the concentrations of the effective materials EM1 and EM2 may be reduced and may vary along the longitudinal direction of the microneedle 220, and thus, concentration gradients may be formed.

Consequently, the concentrations of the effective materials EM1 and EM2 being delivered may be differently set according to the insertion position of the microneedle 220.

In the microneedle patch 200 according to the present disclosure, a curvature is formed in the connection layers 223 or a layer facing and connected to one of the connection layers 223, thus the plurality of layers may be easily separated from each other, the surface area of a curved surface having a certain curvature increases, and consequently, the delivery area of the effective materials EM1 and EM2 may be increased.

The microneedle patch 200 according to another embodiment of the present disclosure has the same configuration, operation principle, and effects as those of the microneedle patch 200 according to the embodiment of the present disclosure, except for the third layer 225 and the connection layer 223B connecting the third layer 225 to the second layer 222, and thus, a related detailed description is omitted.

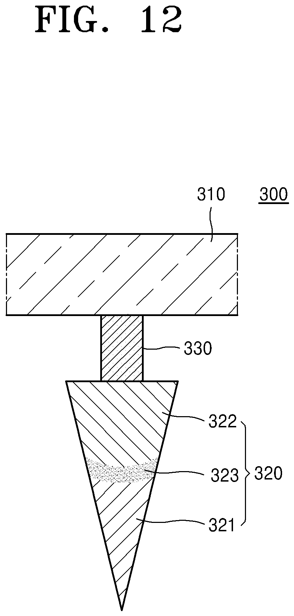

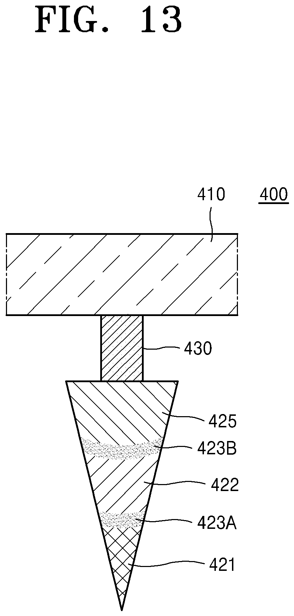

Hereinafter, the configurations, operation principles, and effects of microneedle patches 300 and 400 according to other embodiments of the present disclosure will be described. FIG. 12 is a diagram illustrating the microneedle patch 300 according to another embodiment of the present disclosure. FIG. 13 is a diagram illustrating the microneedle patch 400 according to another embodiment of the present disclosure.

Referring to FIG. 12, the microneedle patch 300 may include a base 310, a microneedle 320, and a shaft 330. The microneedle 320 may include a first layer 321, a second layer 322, and a connection layer 323, and may have a layered structure.

The microneedle 320 may be the same as the microneedle 120 described above. As described above, the microneedle 320 may precisely deliver an effective material to a target position. The shaft 330 may connect the base 310 to the microneedle 320.

The shaft 330 may extend a certain distance in the longitudinal direction of the microneedle 320. The shaft 330 may allow the microneedle 320 to be deeply inserted. That is, the length of the shaft 330 may allow the effective material of the microneedle 320 to be delivered to a deep position under the skin of a user.

The shaft 330 may decompose in vivo to easily separate the base 310 and the microneedle 320 from each other. Because the volume of the shaft 330 is smaller than that of the microneedle 320, the shaft 330 may be dissolved in vivo earlier than is the microneedle 320.

After the shaft 330 is dissolved, the microneedle 320 remains inserted in the skin of the user, and the base 310 may be easily removed. The shaft 330 may decompose in vivo faster than the microneedle 320. The base material of the shaft 330 may be formed of a material that decomposes in vivo faster than the microneedle 320.

Thus, when the microneedle patch 300 is inserted into the skin of the user, the shaft 330 may be rapidly dissolved, and the base 310 may be easily removed.

Although FIG. 12 illustrates that the microneedle 320 consists of the first layer 321, the second layer 322, and the connection layer 323, but the present disclosure is not limited thereto, and various modifications are possible, for example, as illustrated in FIG. 13, a first layer 421, a second layer 422, a third layer 425, and connection layers 423A and 423B formed between the first layer 421 and the second layer 422, and between the second layer 422 and the third layer 425, respectively, may be included.

In the microneedle patches 300 and 400 according to other embodiments of the present disclosure, the bases 310 and 410 and the microneedles 320 and 420 have the same configurations, operation principles, and effects as those of the bases 110 and 210 and the microneedles 120 and 220 of the microneedle patches 100 and 200 according to the above-described embodiments of the present disclosure, except that the shafts 330 and 430 are arranged between the bases 310 and 410 and the microneedles 320 and 420, respectively, and thus, a related detailed description is omitted.

Hereinafter, a method of manufacturing a microneedle patch according to an embodiment of the present disclosure will be described.

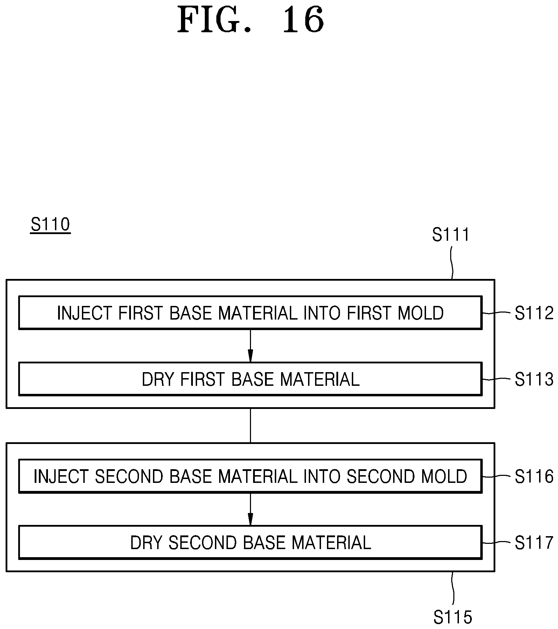

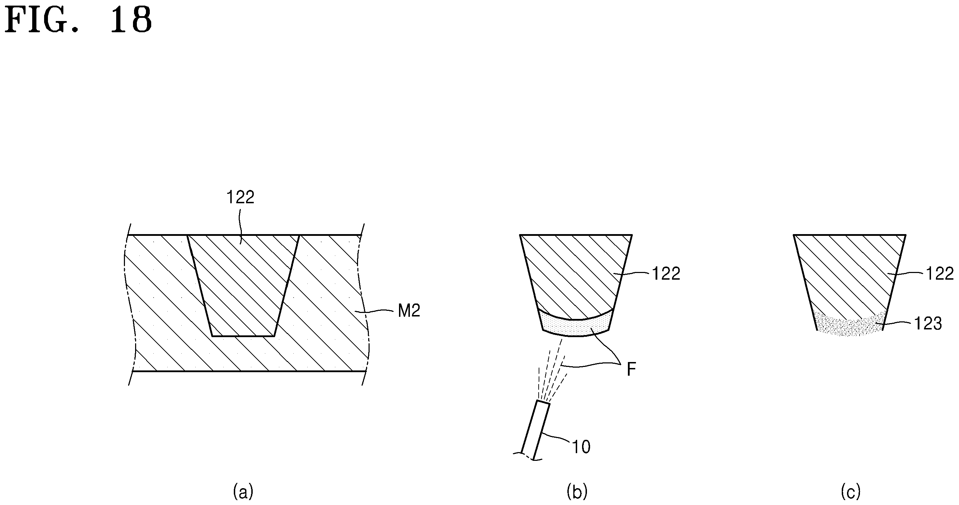

FIG. 16 is a flowchart of operation S110 of forming a plurality of layers, according to an embodiment of the present disclosure. FIGS. 17 and 18 are diagrams illustrating processes of forming connection layers. FIG. 3 is a diagram illustrating a portion of a microneedle patch.

Referring to FIGS. 14 to 16, the method of manufacturing the microneedle patch 100 according to an embodiment of the present disclosure may include forming a microneedle (S100) and connecting a base to the microneedle (S200).

In the forming of the microneedle (S100), the microneedle 120, which contains the effective material EM and includes a plurality of layers, is formed, and operations S100 may include forming the plurality of layers (S110), spraying a fluid onto at least one of the plurality of layers (S120), and connecting the plurality of layers to each other (S130).

Referring to FIGS. 1, 2, and 14 to 16, according to an embodiment of the present disclosure, the microneedle 120 manufactured in the forming of the microneedle (S100) may have a multi-layer structure and may include the first layer 121, the second layer 122, and the connection layer 123.

The microneedle 120 according to an embodiment of the present disclosure may be formed of a biocompatible material and an additive.

The biocompatible material may include at least any one of CMC, HA, alginic acid, pectin, carrageenan, chondroitin sulfate, dextran sulfate, chitosan, polylysine, carboxymethyl chitin, fibrin, agarose, pullulan, polyanhydride, polyorthoester, polyetherester, polyesteramide, polybutyric acid, polyvaleric acid, polyacrylate, an ethylene-vinyl acetate polymer, acryl-substituted cellulose acetate, polyvinyl chloride, polyvinyl fluoride, polyvinyl imidazole, chlorosulphonate polyolefins, polyethylene oxide, PVP, HPMC, EC, HPC, cyclodextrin, maltose, lactose, trehalose, cellobiose, isomaltose, turanose, and lactulose, or at least any one of a copolymer of monomers forming such polymers and cellulose.

The additive may include at least any one of trehalose, oligosaccharide, sucrose, maltose, lactose, cellobiose, HA, alginic acid, pectin, carrageenan, chondroitin sulfate, dextran sulfate, chitosan, polylysine, collagen, gelatin, carboxymethyl chitin, fibrin, agarose, PVP, PEG, polymethacrylate, HPMC, EC, HPC, carboxymethyl cellulose, cyclodextrin, gentiobiose, cetrimide (alkyltrimethylammonium bromide), cetrimonium bromide (hexadecyltrimethylammonium bromide (CTAB)), gentian violet, benzethonium chloride, docusate sodium salt, a SPAN-type surfactant, polysorbate (Tween), sodium lauryl sulfate (SDS), benzalkonium chloride, and glyceryl oleate.

The term "hyaluronic acid (HA)" is used herein to encompass hyaluronic acid, hyaluronates (e.g., sodium hyaluronate, potassium hyaluronate, magnesium hyaluronate, and calcium hyaluronate), and mixtures thereof. The term "hyaluronic acid (HA)" is also used here to encompass cross-linked hyaluronic acid and/or non-cross-linked hyaluronic acid.

According to an embodiment of the present disclosure, the molecular weight of the HA is 2 kDa to 5000 kDa.

According to another embodiment of the present disclosure, the molecular weight of the HA is 100 kDa to 4500 kDa, 150 kDa to 3500 kDa, 200 kDa to 2500 kDa, 220 kDa to 1500 kDa, 240 kDa to 1000 kDa, or 240 kDa to 490 kDa.

The CMC used herein may be known CMC with various molecular weights. For example, the average molecular weight of the CMC used herein is 90,000 kDa, 250,000 kDa, or 700,000 kDa.

The disaccharides may be sucrose, lactulose, lactose, maltose, trehalose, cellobiose, or the like, and may particularly include sucrose, maltose, and trehalose.

In an alternative embodiment, the microneedle 120 may include an adhesive. The adhesive is at least one adhesive selected from the group consisting of silicone, polyurethane, HA, a physical adhesive (Gecko), polyacryl, EC, hydroxymethyl cellulose, ethylene-vinyl acetate, and polyisobutylene.

In an alternative embodiment, the microneedle 120 may further include a metal, a polymer, or an adhesive.

The microneedle 120 may include an effective material EM. The microneedle 120 may include the effective material EM in at least a portion thereof, and the effective material EM may be a pharmaceutically, medically, or cosmetically effective material.

For example, the effective material EM may include, but is not limited to, a protein/peptide medicine, and may include at least one of a hormone, a hormone analogue, an enzyme, an enzyme inhibitor, a signal transduction protein or a portion thereof, an antibody or a portion thereof, a single-chain antibody, a binding protein or a binding domain thereof, an antigen, an adherent protein, a structural protein, a regulatory protein, a toxic protein, a cytokine, a transcription regulator, a blood coagulation factor, and a vaccine.

In detail, the protein/peptide medicine may include at least one of insulin, IGF-1, growth hormone, erythropoietin, G-CSFs, GM-CSFs, interferon alpha, interferon beta, interferon gamma, interleukin-1 alpha and beta, interleukin-3, interleukin-4, interleukin-6, interleukin-2, EGFs, calcitonin, ACTH, TNF, atobisban, buserelin, cetrorelix, deslorelin, desmopressin, dynorphin A (1-13), elcatonin, eleidosin, eptifibatide, GHRH-II, gonadorelin, goserelin, histrelin, leuprorelin, lypressin, octreotide, oxytocin, pitressin, secretin, sincalide, terlipressin, thymopentin, thymosine, triptorelin, bivalirudin, carbetocin, cyclosporine, exedine, lanreotide, LHRH, nafarelin, parathyroid hormone, pramlintide, enfuvirtide (T-20), thymalfasin, and ziconotide.

In addition, the effective material EM may be a cosmetic material such as a skin lightening agent, a filler, a wrinkle reducing agent, or an antioxidant.

In an embodiment, the effective material EM may be colloid particles dispersed in a solvent forming the microneedle 120. The particles themselves may be the effective material EM or may include a coating material carrying the effective material EM.

The effective material EM may be intensively distributed in a partial layer of the microneedle 120. That is, the effective material EM may be at a certain height in the microneedle 120, and thus, the effective material EM may be effectively delivered.

In another embodiment, the effective material EM may be dissolved in the microneedle 120. The effective material EM may be dissolved in the base material of the microneedle 120, such as the biodegradable materials described above, to constitute the microneedle 120.

The effective material EM may be uniformly dissolved in the base material and may be intensively distributed at a certain height of the microneedle 120, like the above-described particles.

According to an embodiment of the present disclosure, in the microneedle 120 manufactured in the forming of the microneedle (S100), the concentration of the effective material EM may vary along the longitudinal direction (the vertical direction in FIG. 2) of the microneedle 120, and a concentration gradient may be formed. This will be described in detail below.

According to an embodiment of the present disclosure, the forming of the microneedle (S100) may include forming a plurality of layers (S110), spraying a fluid onto at least one of the plurality of layers (S120), and connecting the plurality of layers to each other (S130).

Referring to FIGS. 14 to 16, according to an embodiment of the present disclosure, in the forming of the plurality of layers (S110), the first layer 121 and the second layer 122 constituting the microneedle 120 having a multi-layer structure may be provided.

The microneedle 120 manufactured by performing the method for manufacturing a microneedle patch according to an embodiment of the present disclosure may have a two-layer structure including the first layer 121 and the second layer 122, but the present disclosure is not limited thereto, and various modifications are possible, for example, the microneedle 120 may have a three-layer structure including the first layer 221, the second layer 222, and the third layer 225 as illustrated in FIG. 9.

Hereinafter, the configuration of the microneedle 120 including the first layer 121, the second layer 122, and the connection layer 123 connecting the first layer 121 to the second layer 122 will be mainly described.

Referring to FIG. 16, the forming of the plurality of layers (S110) according to an embodiment of the present disclosure may include forming a first layer (S111) and forming a second layer (S 115).

Referring to FIG. 16, the forming of the first layer (S111) according to an embodiment of the present disclosure may include injecting a first base material into a first mold (S112) and drying the first base material (S113).

Referring to FIG. 17, the first layer 121 according to an embodiment of the present disclosure may be formed by injecting the first base material into a first mold M1 and drying the first base material. In detail, referring to (a) of FIG. 17, in the injecting of the first base material into the first mold (S112), the first base material for forming the first layer 121 may be injected into the first mold M1 in which a groove portion is formed.

The groove portion formed in the first mold M1 may be formed such that the cross-sectional area thereof with respect to the central axis in the longitudinal direction decreases toward the lower side (based on the direction illustrated in (a) of FIG. 17), and may be formed in a conical shape such that the sharpened tip ST of the first layer 121 may be formed.

The upper surface of the first layer 121 opposite to one side (the lower side in (a) of FIG. 17) of the first layer 121 where the sharpened tip ST is formed may be formed to have a certain curvature and to be convex toward the sharpened tip ST.

In the drying of the first base material (S113), a process of drying the first base 110 injected into the first mold M1 is performed. The upper surface (based on the direction as illustrated in (a) of FIG. 17) of the first layer 121 may have the certain curvature and be formed to be convex toward the sharpened tip ST, due to the viscosity and drying-induced shrinkage of the first base material.

Referring to FIG. 15 and (a) of FIG. 17, in the spraying of the fluid onto the at least one of the plurality of layers (S120), the fluid F may be sprayed onto the dried first layer 121 or second layer 122.

(a) of FIG. 17 illustrates that the fluid F is sprayed onto the first layer 121, and the fluid F may be sprayed from the nozzle 10 arranged outside the first mold M1, and in detail, the fluid F may include moisture.

As illustrated in (a) of FIG. 17, in the spraying of the fluid F, the sprayed fluid F may dissolve the first layer 121. As the fluid F dissolves a certain region of an upper portion of the first layer 121, the connection layer 123 may be formed.

According to an embodiment of the present disclosure, the fluid F sprayed onto the first layer 121 to form the connection layer 123 includes moisture, but is not limited thereto, and various modifications are possible, for example, the fluid F may include various materials capable of forming a concentration gradient such that the fluid F is sprayed onto the first layer 121 and then dissolves the first layer 121 to cause the concentration of the effective material EM contained in the first layer 121 to vary along the longitudinal direction of the first layer 121.

Referring to (b) of FIG. 17, the connection layer 123, which is integrally formed with and connected to the first layer 121 by dissolving the upper surface of the first layer 121, may be formed such that the side (the upper side in (b) of FIG. 17) opposite to the side (the lower side in (b) of FIG. 17) connected to the first layer 121 is convex toward the first layer 121.

For example, the connection layer 123 may have the same curvature as that of the first layer 121 and may have a first curved surface, which is on the upper surface thereof and convex toward the sharpened tip ST.

Referring to FIG. 3, according to an embodiment of the present disclosure, one surface (the lower surface in FIG. 3) of the second layer 122 facing the connection layer 123 may have a certain curvature and may be provided with a second curved surface.

The first curved surface formed on the upper surface of the connection layer 123, which is integrally formed with the first layer 121 by dissolving a certain region of the first layer 121, and the second curved surface formed on the lower surface of the second layer 122 may have the same curvature and be in contact with each other.

Referring to FIG. 3, according to an embodiment of the present disclosure, the first point PK1 may be positioned at the center of the central axis of the longitudinal direction (the vertical direction in FIG. 3) of the microneedle 120 and on the connection layer 123, and the second point PK2 may be positioned at the outer side of the connection layer 123 in the radial direction with respect to the first point PK1.

Referring to FIG. 3, the distance from the sharpened tip ST formed at one side (the lower side in FIG. 3) of the first layer 121 to the first point PK1 may be less than the distance from the sharpened tip ST to the second point PK2.

Referring to FIG. 3, according to an embodiment of the present disclosure, the upper surface (based on the direction as illustrated in FIG. 3) of the connection layer 123 has a certain curvature and is formed to be convex toward the sharpened tip ST formed in the first layer 121, thus, a region where the connection layer 123 and the second layer 122 are connected to each other may be thin along the edge, and the first layer 121 and the second layer 122 may be easily separated from each other.

Referring to (b) of FIG. 17, according to an embodiment of the present disclosure, the spraying of the fluid onto the at least one of the plurality of layers (S120) includes changing the concentration of the effective material in which the connection layer 123 connected to the first layer 121 by dissolving it causes the concentration of the effective material EM contained in the first layer 121 to relatively decrease in the direction from the sharpened tip ST to the connection layer 123.

Consequently, the concentration of the effective material EM contained in the first layer 121 may be set to vary along the longitudinal direction (the vertical direction in FIG. 2) of the microneedle 120, specifically, of the first layer 121, and in detail, the concentration of the effective material EM may relatively decrease in the direction away from the sharpened tip ST.

That is, the microneedle 120 having a concentration gradient may be manufactured such that the concentration of the effective material EM varies along the longitudinal direction of the microneedle 120 due to the connection layer 123.

In addition, when, after the first layer 121 is formed, the connection layer 123 dissolves an upper region of the first layer 121 and thus be in contact with a certain region of the second layer 122, specifically, a lower region of the second layer 122 facing the upper region of the first layer 121, the connection layer 123 may dissolve the lower region to adhesively connect the first layer 121 to the second layer 122.

In a conventional method of manufacturing a microneedle having a plurality of layers, a first layer is formed by injecting and drying a first base material into a single mold, and a second layer is formed by injecting and drying a second base material onto the first layer, whereas, in the method of manufacturing a microneedle patch according to an embodiment of the present disclosure, different layers are formed in respective molds, then the fluid F is sprayed onto any one of the plurality of layers facing each other to form the connection layer 123, and then the plurality of layers are connected to each other, and accordingly, the period of time required for manufacturing the microneedle 120 may be reduced, and the productivity of the microneedle patch 100 having the microneedle 120 may be improved.

Referring to FIG. 16 and (a) of FIG. 18, the forming of the second layer (S115) by injecting and drying the second base material may include injecting the second base material into a second mold (S116) and drying the second base material (S117).

Referring to (a) of FIG. 18, a second mold M2 may be provided with a groove portion corresponding to a preset shape of the second layer 122.

In detail, the groove portion may be formed such that the cross-sectional area thereof with respect to the central axis in the longitudinal direction relatively decreases in the downward direction along the longitudinal direction (the vertical direction in (a) of FIG. 18).

In an alternative embodiment, the groove portion formed in the second mold M2 may be flat so as to be in contact with the first layer 121 or the connection layer 123 integrally formed with and connected to the first layer 121.

The second layer 122 according to an embodiment of the present disclosure does not contain the effective material EM, but the present disclosure is not limited thereto, and various modifications are possible, for example, the second layer 122 may include an effective material different from the effective material EM contained in the first layer 121.

Referring to FIG. 16 and (a) of FIG. 18, after the second base material is injected into the second mold M2, the second base material is dried (S117). When the second base material is completely dried, the second layer 122 may be formed, and may be withdrawn from the second mold M2.

Referring to (b) of FIG. 18, in an alternative embodiment, in the spraying of the fluid onto the at least one of the plurality of layers (S120), the fluid F may be sprayed onto one surface (the lower surface in (b) of FIG. 18) of the second layer 122 dried and withdrawn from the second mold M2.

In the spraying of the fluid onto the at least one of the plurality of layers (S120), the fluid F may be sprayed onto at least one of the first layer 121 and the second layer 122.

As illustrated in (a) of FIG. 17, after the fluid F is sprayed onto the upper surface of the first layer 121, the connection layer 123 is formed, and the second layer 122 formed and withdrawn from the second mold M2 may be in contact with and connected to the connection layer 123.

In an alternative embodiment, referring to (b) of FIG. 18, the fluid F may be sprayed onto one surface (the lower surface in (b) of FIG. 18) of the second layer 122 dried and withdrawn from the second mold M2. The fluid F may be sprayed from the nozzle 10 arranged outside the second layer 122, and in detail, the fluid F may include moisture.

Referring to (c) of FIG. 18, the fluid F sprayed onto one surface of the second layer 122 may dissolve one surface (the lower surface in (c) of FIG. 18) of the second layer 122, and the connection layer 123 may be formed.

The fluid F sprayed onto the second layer 122 dissolves a certain region of the lower surface of the second layer 122, and the connection layer 123 connected to and integrally formed with the second layer 122 may be formed such that the side opposite to one side connected to the second layer 122 is convex toward the first layer 121.

The connection layer 123, which is integrally formed with the second layer 122 by dissolving a certain region of the second layer 122, may be connected to the first layer 121.

In this case, the connection layer 123 may connect the first layer 121 to the second layer 122 by dissolving the upper surface of the first layer 121.

In an alternative embodiment, the fluid F may be sprayed onto both the first layer 121 and the second layer 122, then dissolve certain regions of the first layer 121 and the second layer 122, and connect the first layer 121 to the second layer 122.

The fluid F is sprayed onto the first layer 121 and the second layer 122, respectively, to form the connection layer 123 by dissolving a certain upper region of the first layer 121 and dissolving a certain lower region of the second layer 122, and in the connecting of the plurality of layers to each other (S130), the connection layer 123 may connect the first layer 121 to the second layer 122, and the connection layer 123 formed by dissolving the certain regions of the first layer 121 and the second layer 122 may stably and adhesively connect the first layer 121 to the second layer 122.

As the fluid F is sprayed onto the first layer 121 and the second layer 122 to form the connection layer 123, the concentration of the effective material is changed such that the concentration varies along the longitudinal direction of the microneedle 120.

When the effective material EM is contained in the first layer 121, the concentration of the effective material EM may relatively decrease in the direction away from the sharpened tip ST (in the downward direction in (b) of FIG. 17) due to the formation of the connection layer 123.

In an alternative embodiment, when the effective material EM is contained in the second layer 122, the concentration of the effective material EM may relatively decrease toward the first layer 121 due to the formation of the connection layer 123.

That is, as the connection layer 123 dissolves the certain regions of the first layer 121 and the second layer 122 to connect them to each other, the concentration of the effective material EM varies along the longitudinal direction of the microneedle 120, and a concentration gradient may be formed.

Accordingly, the concentration of the effective material EM that may be delivered along the longitudinal direction of the microneedle 120 penetrating into the body of the user may be adjusted, and the amount of the effective material EM delivered into the body of the user at a corresponding position may be reduced by the connection layer 123 formed in a certain section.

In addition, in the conventional method, the first layer 121 is formed by injecting and drying the first base material into a single mold, and then the second layer 122 is formed by injecting and drying the second base material onto the first layer 121, whereas, in the method according to the present disclosure, the first layer 121 and the second layer 122 are formed in different molds, respectively, the fluid F is sprayed onto at least one of the first layer 121 and the second layer 122 to form the connection layer 123, and then the first layer 121 and the second layer 122 are connected to each other, and accordingly, the period of time required for manufacturing of a microneedle patch may be reduced.

Referring to FIG. 14, according to an embodiment of the present disclosure, in the connecting of the base to the microneedle (S200), the base 110 may be connected to one surface of the microneedle 120.

The base 110 according to an embodiment of the present disclosure supports the microneedle 120, and one surface of the base 110 may be in contact with the skin of the user and the other surface may be exposed to the outside.

The base 110 according to an embodiment of the present disclosure may be removed after the microneedles 120 are inserted into the skin. In detail, the base 110 may be removed from the skin by the user applying a force.

In an alternative embodiment, a portion at which the base 110 and the microneedle 120 are coupled to each other first dissolves, and thus the base 110 may be removed after a certain period of time has elapsed after the microneedle patch is attached to the skin.

In another alternative embodiment, the base 110 may dissolve after a long period of time has elapsed after the microneedle patch is attached to the skin. In an alternative embodiment, the base 110 to be attached to the skin of the user may be formed of a dissolvable material, and may be removed by the user applying a material for dissolution on the base 110, if necessary.

The base 110 according to an embodiment of the present disclosure may include any one of materials included in the microneedle 120. The base 110 may include a biodegradable material similarly to the microneedle 120.

For example, the base 110 may include the same material as that of any one of a plurality of layers of the microneedle 120.

In an alternative embodiment, the base 110 may include a physiologically active substance. After attaching the microneedle patch according to an embodiment of the present disclosure to the skin, an effective drug may be effectively delivered to the patient by the physiologically active substance released from the base 110.

In addition, the base 110 and the microneedles 120 may be easily separated from each other by the physiologically active substance released from the base 110.

The base 110 according to an embodiment of the present disclosure may have a property of dissolving later than does the closest layer of the microneedle 120, i.e., a layer that is farthest away from a tip formed at the lower side of the microneedle 120, specifically, a sharpened tip ST of the microneedle 120.

Consequently, a portion of the microneedle 120, which is adjacent to the base 110, dissolves the fastest, and thus the base 110 may be easily separated from the microneedle 120.

In an alternative embodiment, the base 110 may include a water-soluble polymer. The base 110 may be formed of a water-soluble polymer and may include other additives (e.g., disaccharides, etc.). In addition, it is preferable that the base 110 does not include a drug or the effective material EM.

The base 110 according to an embodiment of the present disclosure may include a biocompatible material. A biocompatible material selected as a base material of the microneedle 120 may also be selected as a base material of the base 110.

FIG. 7 is a diagram illustrating a process in which the microneedle patch 100 manufactured by the method of manufacturing a microneedle patch according to an embodiment of the present disclosure is attached to the skin of a user and then a drug is delivered, wherein the microneedle patch 100 is attached to the skin and then the layers of the microneedle 120 biodegrade to deliver the drug.

Although FIG. 7 illustrates the effective material EM is included in the first layer 121 and delivered to the dermis DEM, the effective material EM may be included in the second layer 122, in which case, the effective material EM may be delivered to the epidermis EPM.

Referring to (a) of FIG. 7, the microneedle patch 100 is attached to the skin. The microneedle 120 may be inserted into the skin, and then the base 110 may cover the top of the skin.

Referring to (b) of FIG. 7, the microneedle 120 may biodegrade within the skin. The microneedle 120 may be inserted into the skin, and then the base 110 may cover the top of the skin.

Referring to (c) of FIG. 7, the effective material EM may be released from the microneedle 120. When the first layer 121 begins to biodegrade, the effective material EM included therein may be delivered to the dermis DEM.

Referring to FIG. 7, according to an embodiment of the present disclosure, the connection layer 123 may be arranged between the first layer 121 and the second layer 122, and connect the first layer 121 to the second layer 122, and as the connection layer 123 adhesively connects the first layer 121 to the second layer 122 by dissolving at least one of the first layer 121 and the second layer 122, a section in which the concentration of the effective material EM relatively decreases along the longitudinal direction (the vertical direction in FIG. 7) of the microneedle 120, specifically, a concentration gradient, may be formed in the connection layer 123.

In detail, the concentration of the effective material EM included in the first layer 121 may relatively decrease in the direction from one side (the lower side in FIG. 7) where the sharpened tip ST is formed, to the upper side. Accordingly, the microneedle 120 may be inserted into the body of the user, and the concentration of the effective material EM may be adjusted according to the depth.

Although not shown in FIG. 7, in an alternative embodiment, an effective material may be included in the second layer 122, and when the connection layer 123 is connected to the first layer 121 by dissolving a certain region of the second layer 122 to connect the first layer 121 to the second layer 122, the concentration of the effective material included in the second layer 122 may relatively decrease in the direction from the base 110 to the first layer 121, and a concentration gradient may be formed.

Referring to FIG. 8, the method of manufacturing a microneedle patch according to an embodiment of the present disclosure may further include forming the coating layer 124. The microneedle 120 may include the first layer 121, the second layer 122, and the connection layer 123, and the coating layer 124 may be arranged on the outer side of the microneedle 120.

After the forming of the microneedle (S100) by spraying the fluid F onto at least one of the plurality of layers, specifically, the first layer 121 and the second layer 122, and forming the connection layer 123 to adhesively connect the first layer 121 to the second layer 122, the formed microneedle 120 may be dipped in a coating solution to form the coating layer 124.

The coating layer 124 may be formed of a biocompatible polymer. The coating layer 124 may decompose after inserted into the skin.

In an alternative embodiment, the coating layer 124 may be formed of a biocompatible polymer. The coating layer 124 may decompose when inserted into the skin.

In an alternative embodiment, the coating layer 124 may include a physiologically active substance. When the coating layer 124 is inserted into the skin, the coating layer 124 may be activated first before the effective material EM is injected, and thus, the delivery effectiveness the effective material EM may be increased.

In an alternative embodiment, the coating layer 124 may be formed of a material having a high biodegradation rate. The coating layer 124 may be formed of a material having a biodegradation rate greater than those of the first layer 121, the second layer 122, and the connection layer 123, and thus, the in vivo decomposition rate of the coating layer 124 may be greater than those of the first layer 121, the second layer 122, and the connection layer 123.

In an alternative embodiment, the coating layer 124 may be formed of a material having a low biodegradation rate. The coating layer 124 may be formed of a material having a biodegradation rate less than those of the first layer 121, the second layer 122, and the connection layer 123, and thus, the in vivo decomposition rate of the coating layer 124 may be less than those of the first layer 121, the second layer 122, and the connection layer 123.

According to an embodiment of the present disclosure, after the microneedle 120 is inserted into the skin, a drug may be delivered after a certain period of time has elapsed, and thus the effective material EM may be delivered into the body at a preferred appropriate point of time.

In an alternative embodiment, the coating layer 124 may increase the stiffness of the microneedle 120. Because the coating layer 124 covers the outer side of the connection layer 123 connected to the first layer 121 and the second layer 122, the first layer 121 and the second layer 122 may be prevented from being separated from each other when the microneedle 120 is inserted into the skin.

The formation of the coating layer 124 according to an embodiment of the present disclosure may be performed between the forming of the microneedle (S100) and the connecting of the base to the microneedle (S200). However, the present disclosure is not limited thereto, and various modifications are possible, for example, the coating layer 124 may be formed after the connecting of the base to the microneedle (S200).

According to an embodiment of the present disclosure, after the microneedle 120 is inserted into the skin, a drug may be delivered after a certain period of time has elapsed, and thus the effective material EM may be delivered at a preferred appropriate point of time.

In an alternative embodiment, the coating layer 124 may increase the stiffness of the microneedle 120. Because the coating layer 124 covers the outer side of the connection layer 123 connected to the first layer 121 and the second layer 122, the first layer 121 and the second layer 122 may be prevented from being separated from each other when the microneedle 120 is inserted into the skin.

Referring to FIG. 9, in the method of manufacturing a microneedle patch according to another embodiment of the present disclosure, three layers may be formed in the forming of the plurality of layers (S110).

The three layers may be formed by injecting and drying respective base materials into different molds, a connection layer may be formed in the spraying of the fluid onto the at least one of the plurality of layers (S120), and a concentration gradient may be formed as the concentration of the effective material is changed in a region where the connection layer is formed.

Referring to FIG. 9, in the spraying of the fluid onto the at least one of the plurality of layers (S120), the connection layer 223A may be formed by spraying the fluid F onto at least one of the first layer 221 and the second layer 222, and the connection layer 223B may be formed by spraying the fluid F onto at least one of the second layer 222 and the third layer 225.

Referring to FIG. 9, the fluid F is sprayed onto the upper surfaces of the first layer 221 and the second layer 222 to dissolve certain upper regions of the first layer 221 and the second layer 222 and thus form the connection layers 223A and 223B, respectively, and the upper surface of the connection layer 223A facing the second layer 222 may have the first curvature RA and may be formed to be convex toward the first layer 221.

In addition, the upper surface of the connection layer 223B facing the third layer 225 may have the second curvature RB and may be formed to be convex toward the second layer 222.

The first curvature RA and the second curvature RB may be equal to each other. However, the present disclosure is not limited thereto, and various modifications are possible, for example, the first curvature RA and the second curvature RB may be different from each other.

Referring to FIG. 9, each of the connection layers 223A and 223B may be formed to have both sides symmetrical to each other with respect to the longitudinal direction of the microneedle 220.

Referring to FIG. 9, the first effective material EM1 may be included in the first layer 221, and the second effective material EM2 may be included in the second layer 222.

The connection layer 223A, which is integrally formed with the first layer 221 by dissolving a certain region of the first layer 221, may contain the first effective material EM1, and may have a concentration gradient such that the concentration of the first effective material EM1 relatively decreases in the direction from the sharpened tip ST of the first layer 221 to the second layer 222.

Referring to FIG. 9, the connection layer 223B, which is integrally formed with the second layer 222 by dissolving a certain region of the second layer 222, may contain the second effective material EM2, and may have a concentration gradient such that the concentration of the second effective material EM2 relatively decreases in the direction from the first layer 221 to the third layer 225.

Accordingly, the concentration gradients may be formed in the connection layers 223A and 223B, respectively, such that the concentrations of the effective materials vary along the longitudinal direction of the microneedle 220, and the effective materials may be included at respective concentrations adjusted along the longitudinal direction of the microneedle 220.

In addition, a plurality of layers may be formed in respective molds without injecting and drying a base material to form one layer and injecting and drying a base material for forming another layer thereon, and the fluid F may be sprayed onto at least one of a pair of stacked layers to dissolve a certain region to adhesively connect the pair of layers to each other, and accordingly, the period of time required for manufacturing the microneedle patch including the microneedle 220 and the base 210 connected to the microneedle 220 may be reduced.

Referring to FIG. 12, in the method of manufacturing a microneedle patch according to another embodiment of the present disclosure, the microneedle patch 300 may include the base 310, the microneedle 320, and the shaft 330. The microneedle 320 may include the first layer 321, the second layer 322, and the connection layer 323, and may have a layered structure.

However, the present disclosure is not limited thereto, and various modifications are possible, for example, the microneedle patch 200 may have a layered structure including three or more layers.

Referring to FIG. 12, the shaft 330 may connect the base 310 to the microneedle 320. Although not shown in the drawings, the method of manufacturing a microneedle patch according to another embodiment of the present disclosure may further include connecting the base 310 to the microneedle 320 through the shaft 330.

The connecting may be performed after the forming of the microneedle (S100), and the shaft 330 may be first connected to the microneedle 320 and then to the base 310. However, the present disclosure is not limited thereto, and various modifications are possible, for example, the shaft 330 may be first connected to the base 310 and then to the microneedle 320.

The connecting of the base 310 to the microneedle 320 through the shaft 330 may include spraying the fluid F onto at least one of the shaft 330 and the microneedle 320.

Referring to FIG. 12, the fluid F may be sprayed onto one surface (the lower surface in FIG. 12) of the shaft 330 facing the second layer 322 of the microneedle 320, and a certain lower region (based on the direction as illustrated in FIG. 12) of the shaft 330 may be dissolved.

The dissolved certain lower region (based on the direction as illustrated in FIG. 12) of the shaft 330 may form a connection layer in the same manner as the formation of the connection layer 323 provided in the microneedle 320, and may connect the shaft 330 to the microneedle 320 when contacting the microneedle 320 facing the connection layer, specifically, the second layer 322.

In an alternative embodiment, a fluid may be sprayed from an external spray device such as the nozzle 10, onto the microneedle 320, specifically, the second layer 322 facing the shaft 330.

The sprayed fluid F may dissolve a certain upper region (based on the direction as illustrated in FIG. 12) of the second layer 322, form a connection layer in the same manner as the formation of the connection layer 323 formed between the first layer 321 and the second layer 322, and connect the shaft 330 to the microneedle 320 when contacting the shaft 330 facing the connection layer.

The connection layer formed by dissolving a certain upper region of (based on the direction as illustrated in FIG. 12) of the second layer 322 may cause the concentration of the effective material contained in the second layer 322 to vary along the longitudinal direction (the vertical direction in FIG. 12) of the microneedle 320, specifically, the second layer 322.

In detail, as the upper region (based on the direction as illustrated in FIG. 12) of the second layer 322 is dissolved by the fluid F, the concentration of the effective material may relatively decrease in the direction from the lower side to the upper side (based on the direction as illustrated in FIG. 12) of the second layer 322.

Accordingly, the concentration of the effective material may vary along the longitudinal direction of the second layer 322, and a concentration gradient may be formed.

In addition, as the concentration gradient is formed along the longitudinal direction of the second layer 322, the concentration of the effective material contained in the second layer 320 may be differently set according to the depth of the patient to which the microneedle 320 is inserted.

In an alternative embodiment, the fluid F sprayed from the spray device such as the nozzle 10 may be sprayed onto one surface (the lower surface in FIG. 12) of the shaft 330 and one surface of the microneedle 320 facing the shaft 330, specifically, one surface (the upper surface in FIG. 12) of the second layer 322, and then dissolve certain regions of the shaft 330 and the second layer 322.

As the certain regions are dissolved, a connection layer may be formed, and the shaft 330 and the microneedle 320, specifically, the second layer 322 may be connected to each other when the shaft 330 and the microneedle 320 contact each other. As described above, when the fluid F forms the connection layer by dissolving the certain upper region (based on the direction as illustrated in FIG. 12) of the second layer 322, the concentration of the effective material contained in the second layer 322 may vary along the longitudinal direction (the vertical direction in FIG. 12) of the second layer 322, the concentration of the effective material of the second layer 322 may relatively decrease in the direction from the first layer 321 to the shaft 330, and a concentration gradient may be formed.

The shaft 330 may extend a certain distance in the longitudinal direction of the microneedle 320. The shaft 330 may allow the microneedle 320 to be deeply inserted.

That is, the length of the shaft 330 may allow the effective material of the microneedle patch 300 to be delivered to a deep position under the skin of the user.

The shaft 330 may decompose in vivo to easily separate the base 310 and the microneedle 320 from each other. Because the volume of the shaft 330 is smaller than that of the microneedle 320, the shaft 330 may be dissolved in vivo earlier than is the microneedle 320.

After the shaft 330 is dissolved, the microneedle 320 remains inserted in the skin of the user, and the base 310 may be easily removed. The shaft 330 may decompose in vivo faster than the microneedle 320. The base material of the shaft 330 may be formed of a material that decomposes in vivo faster than the microneedle 320.

Thus, when the microneedle patch 300 is inserted into the skin of the user, the shaft 330 may be rapidly dissolved, and the base 310 may be easily removed.

The method of manufacturing a microneedle patch according to another embodiment of the present disclosure includes the same operations as those of the method of manufacturing a microneedle patch according to the embodiment of the present disclosure including forming a microneedle and connecting a base to the microneedle, except for connecting the base 310 to the microneedle 320 through the shaft 330, and thus, a related detailed description is omitted.

In forming a microneedle including a plurality of layers, the method of manufacturing a microneedle patch according to the present disclosure is capable of reducing the period of time required for manufacturing the microneedle patch including the microneedle and a base connected to the microneedle, by individually forming the plurality of layers and then spraying a fluid onto at least one of a pair of layers connected to each other to form a connection layer and thus adhesively connect the plurality of layers to each other, rather than sequentially forming the plurality of layers.

In addition, as the connection layer is integrally formed with a layer including an effective material by dissolving a certain region of the layer, the concentration of the effective material in the region where the connection layer is formed may be relatively low, the concentration of the effective material may vary along the longitudinal direction of the microneedle, and thus a concentration gradient may be formed.

In addition, as the concentration gradient is formed along the longitudinal direction of the microneedle, the manufactured microneedle patch may deliver the effective material to any one of an epidermis, a dermis, subcutaneous fat, and muscle at an appropriate concentration according to the position at which the effective material is activated.

Although the present disclosure has been described with reference to the embodiments illustrated in the drawings, they are merely exemplary, and it will be understood by one of skill in the art that various modifications and equivalent embodiments may be made therefrom. Therefore, the true technical protection scope of the present disclosure should be determined by the appended claims.

The particular implementations shown and described herein are illustrative examples of embodiments and are not intended to otherwise limit the scope of embodiments in any way. Moreover, no item or component is essential to the practice of the present disclosure unless the item or component is specifically described as "essential" or "critical".

The term "the" and other demonstratives similar thereto in the descriptions of embodiments (especially in the following claims) should be understood to include a singular form and plural forms. Furthermore, recitation of ranges of values herein are merely intended to serve as a shorthand method of referring individually to each separate value falling within the range, unless otherwise indicated herein, and each separate value is incorporated into the specification as if it were individually recited herein. Finally, the operations of all methods described herein may be performed in any suitable order unless otherwise indicated herein or otherwise clearly contradicted by context. The present disclosure is not limited to the described order of the operations. The use of any and all examples, or exemplary language (e.g., "and the like") provided herein, is intended merely to better illuminate embodiments and does not pose a limitation on the scope of the embodiments unless otherwise claimed. In addition, various modifications, combinations, and adaptations will be readily apparent to those skilled in this art without departing from the following claims and equivalents thereof.

### INDUSTRIAL APPLICABILITY

The present disclosure provides a microneedle patch. In addition, embodiments of the present disclosure may be applied to patches to be attached to skin for delivering a drug.

## Claims

1. A microneedle patch comprising:
a base; and
a microneedle, which contains an effective material, protrudes from a surface of the base, and comprises a plurality of layers, a concentration of the effective material varying along a longitudinal direction of the microneedle.

2. The microneedle patch of claim 1, wherein the microneedle comprises:
a first layer having a sharpened tip arranged on one side thereof and a surface formed at another side thereof to face the base;
a second layer, which is connected to the base and arranged between the base and the first layer; and
a connection layer, which is arranged between the first layer and the second layer and connects the first layer to the second layer.

3. The microneedle patch of claim 2, wherein the connection layer is integrally formed with the first layer by dissolving the first layer.

4. The microneedle patch of claim 2, wherein the connection layer is integrally formed with the second layer by dissolving the second layer.

5. The microneedle patch of claim 3, wherein one surface of the connection layer, which is opposite to another surface of the connection layer connected to the first layer, has a curvature.

6. The microneedle patch of claim 5, wherein one surface of the second layer facing the connection layer has a curvature.

7. The microneedle patch of claim 5, wherein the one surface of the connection layer has a plurality of curvatures.

8. The microneedle patch of claim 5, wherein both sides of the curvature are symmetrical to each other with respect to a longitudinal central axis of the microneedle.

9. The microneedle patch of claim 1, wherein at least one of the plurality of layers comprises an in vivo degradable polymer.

10. The microneedle patch of claim 1, further comprising a shaft connecting the base to the microneedle.

11. A method of manufacturing a microneedle patch, the method comprising forming a microneedle containing an effective material, wherein the forming of the microneedle comprises:
forming a plurality of layers; spraying a fluid onto at least one of the plurality of layers; and
connecting the plurality of layers to each other.
